# EUROPEAN PATENT SPECIFICATION

(11) **EP 2 854 717 B1**
(45) Date of publication and mention of the grant of the patent: **30.12.2020**
(21) Application number: 13813865.6
(22) Date of filing: 21.05.2013
(51) Int. Cl.: A61F 2/68

(54) **POWERED LOWER EXTREMITY ORTHOTIC**
ANGETRIEBENE ORTHESE FÜR UNTERE EXTREMITÄT
ORTHÈSE ÉLECTRIQUE D'EXTRÉMITÉ INFÉRIEURE

(30) Priority: 24.05.2012 US 201213480160
(43) Date of publication of application: 08.04.2015
(73) Proprietor: Ekso Bionics, Inc., Richmond, CA 94804 (US); The Regents of the University of California, Oakland, CA 94607-5200 (US)
(72) Inventor: SWIFT, Tim, Clovis, CA 94702 (US); ZOSS, Adam, Brian, Berkeley, CA 94702 (US); STRAUSSER, Katherine, Berekley, CA 94702 (US); ROSA, Matthew, San Francisco, CA 94103 (US); KAZEROONI, Homayoon, Berkeley, CA 94705 (US); FAIRBANKS, Dylan, Miller, Berkeley, CA 94703 (US); PILLAI, Minerva, Vasudevan, Pleasant Hill, CA 94523 (US); SCHWARTZ, Miclas, 37085 Goettingen (DE); LAMBRECHT, Bram Gilbert, Antoon, Sunnyvale, CA 94086 (US); KRUSE, Sebastian, Berkeley, CA 94709 (US)
(74) Representative: Dehns
(86) International application number: PCT/US2013/042004
(87) International publication number: WO 2014/007917

(56) References cited:
- WO-A1-2012/048123
- US-A1- 2010 023 133
- US-A1- 2011 082 566
- US-A1- 2011 166 489
- US-B2- 7 747 409
- US-B2- 8 147 436

## Description

### BACKGROUND OF THE INVENTION

The overall invention relates generally to the controlling the trajectory of an artificial foot. The invention expands on developments in prosthetics, while translating certain developments into the field of human exoskeletons which are orthotic devices attached to users who still retain their limbs. In particular, these human exoskeletons are used by individuals who have limbs that are paralyzed and therefore need the capability of joint motion restored much like in the case of prosthetics.

In recent years, major advancements have been made in the field of prosthetics. For instance, not only are prosthetics now commonly available for customized fit in connection with a wide range of amputations, but the prosthetics themselves can be customized for use as well. Therefore, fitting an amputee with a prosthetic includes not only customization for size, but also variations based on various other factors, particularly the types of activities in which the amputee will be utilizing the prosthetic device.

In connection with above-knee prosthetics, both swing and stance controls must be established. Certainly, swing controls have to accommodate for a greater range of motions, with the potential motions even varying in dependence on the age and activity level of the amputee. In this regard, fluid systems have been employed in the past, often due to their ability to establish relatively consistent motions. However, fluctuations in the speed of movement may be needed as well such that proper control of the fluid system is also needed. Also, it is believed that certain properties of developments in the field of prosthetics can be advantageously translated into other orthotic fields, particularly human exoskeletons. An orthotic device according to the preamble of claim 1 is known from WO 2012/048123.

### SUMMARY OF THE INVENTION

The present invention, which is defined in claim 1, is concerned with a powered, lower extremity orthotic which operates similarly to an above knee prosthetic. Any methods disclosed hereinafter do not form part of the scope of the invention. In accordance with one aspect of the invention, a semi-actuated above knee prosthetic system that is mostly passive in nature in that the system only requires power for locomotion during a portion of a walking cycle. In general, the prosthetic includes a shank link adapted to be coupled to an artificial foot, a knee mechanism connected to the shank link at a position remote from the artificial foot and a thigh link adapted to be attached to an above-knee remaining lower limb of an amputee. The knee mechanism is configured to provide flexion and extension movements of the thigh and shank links relative to each other. In accordance with the invention, the prosthetic is operable in either an actuated mode or an un-actuated mode. In the actuated mode, power is delivered to a torque generator connected to the knee mechanism to cause a forced movement between the thigh and shank links. In the un-actuated mode, a control circuit operates in a non-powered manner to allow operation of the knee mechanism with modulated resistance.

In accordance with a preferred embodiment of the invention, an electric motor is connected to a battery source and employed to drive a hydraulic pump which is part of an overall hydraulic power unit including the torque generator used to regulate the knee mechanism. A signal processor controls the operation of the hydraulic power unit in order to establish the actuated and un-actuated modes based on signals received from a plurality of sensors provided on the above-knee prosthetic. Although the location, number and type of sensors can vary, one preferred embodiment employs a stance sensor capable of identifying a particular part of an artificial foot which is in contact with a support surface (e.g., the ground), while the signal processor selects a desired swing state when the artificial foot leaves the support surface based on an estimated location of the artificial foot with respect to a trunk of the amputee. Knee angle, thigh angle, pressure and other sensors can also be employed for additional control purposes.

With this arrangement, the overall system advantageously employs less electric power than fully powered knees and therefore an amputee can walk much longer for a given battery size. In addition, the above-knee prosthetic of the invention is generally smaller than fully actuated knees. Furthermore, the semi-actuated prosthetic knee reduces necessary hip torque and power that the amputee must physically exert by efficiently creating synchronized torque and power during an effective portion of a walking cycle. Even further, the various sensors provide inputs to the signal processor that effectively maximize the range and type of motions generated for the amputee.

The prosthetic knee of the invention also is controlled in a manner that allows the foot, or more specifically the toe, to track a trajectory through space that is consistent with respect to the ground, rather than simply repeating a knee motion during swing regardless of the orientation of the prosthetic with respect to the ground. This is accomplished by measuring the angle of the user's thigh and using it to derive the current knee angle necessary to describe a defined trajectory. In accordance with the overall invention, in addition to uses with prosthetics, this technique has direct application to other orthotic devices, particularly human exoskeletons.

Additional objects, features and advantages of the invention will become more fully evident below from the following detailed description of preferred embodiments wherein like reference numerals refer to corresponding parts in the various views.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
Figure 1 depicts a semi-actuated prosthetic knee constructed in accordance with a first embodiment of the invention;
Figure 2 is a diagram of a first hydraulic valve circuit of the present invention;
Figure 3 is a diagram of the hydraulic valve circuit of Figure 2, further comprising a first check valve;
Figure 4 is a diagram of the hydraulic valve circuit of Figure 3, further comprising a second controllable valve;
Figure 5 is a diagram of the hydraulic valve circuit of Figure 4, further comprising a second check valve;
Figure 6 is a diagram of an alternative hydraulic valve circuit including a parallel path circuit;
Figure 7 is a diagram of an alternative hydraulic valve circuit including an actuator valve;
Figure 8 is a diagram of the hydraulic valve circuit of Figure 7, further comprising a first check valve;
Figure 9 is a diagram of the hydraulic valve circuit of Figure 8, further comprising a second controllable valve;
Figure 10 is a diagram of the hydraulic valve circuit of Figure 9, further comprising a second check valve;
Figure 11 is a diagram of an alternative hydraulic valve circuit including a parallel path circuit;
Figure 12 is a diagram of an alternative hydraulic valve circuit including a three-way valve;
Figure 13 depicts the three way valve of the hydraulic valve circuit of Figure 12 in use;
Figure 14 is a diagram of the hydraulic valve circuit of Figure 12, further comprising a first check valve;
Figure 15 depicts the three way valve of the hydraulic valve circuit of Figure 14 in use;
Figure 16 is a diagram of an alternative hydraulic valve circuit including a fluid reservoir;
Figure 17 is a diagram of the hydraulic valve circuit of Figure 12, further including a parallel path circuit;
Figure 18 is a diagram of an alternative hydraulic valve circuit including a second three-way valve;
Figure 19 is a diagram of an alternative hydraulic valve circuit including a four-way valve;
Figure 20 is a side view of the semi-actuated prosthetic knee of Figure 1;
Figure 21 is a more detailed perspective view of the semi-actuated prosthetic knee of Figure 20;
Figure 22 is an exploded view of the semi-actuated prosthetic knee of Figure 21;
Figure 23 is a partial perspective view of the hydraulic valve circuit of Figure 16 with fluid flow during an actuated mode in extension;
Figure 24 is a partial perspective view of the hydraulic valve circuit of Figure 16 with fluid flow during an un-actuated mode in extension;
Figure 25 is an exploded view of the power unit in Figure 1;
Figure 26 is an exploded view of the three-way valve of Figure 25;
Figure 27 is a partial cross-sectional side view of the three-way valve of Figure 26 in a first position;
Figure 28 is a partial cross-sectional side view of the three-way valve of Figure 26 in a second position;
Figure 29A is a partial cross-sectional top view of the three-way valve of Figure 26 in a first position;
Figure 29B is a partial cross-sectional top view of the three-way valve of Figure 26 in a second position;
Figure 29C is a partial cross-sectional top view of the three-way valve of Figure 26 in a third position;
Figure 29D is a partial cross-sectional top view of the three-way valve of Figure 26 in a fourth position;
Figure 30 is a partial cross-sectional view of a hydraulic power circuit of the present invention;
Figure 31 is a partial exploded view of the semi-actuated knee of Figure 20;
Figure 32A is a partial cross-sectional back perspective view of a stance sensor of the present invention;
Figure 32B is a back perspective view of the stance sensor of Figure 32A;
Figure 32C is a front perspective view of the stance sensor of Figure 32A;
Figure 33 is a partial exploded view of a semi-actuated prosthetic knee of the present invention;
Figure 34 is a diagram of states implemented by a signal processor in accordance with the invention;
Figure 35 is an electrical schematic showing the connection of an electric power source to a motor controller; and
Figure 36 is a schematic view of an exoskeleton system employing foot trajectory capabilities in accordance with an aspect of the invention;
Figure 37 sets forth a flow chart associated with the system of Figure 36;
Figure 38 is a schematic view of an exoskeleton system, similar to that of Figure 36, in accordance with another embodiment of the invention;
Figure 39 illustrates an exemplary trajectory operation for the exoskeleton system of Figure 38;
Figures 40A and 40B illustrates side and rear views respectively, of another embodiment of the exoskeleton system of the invention;

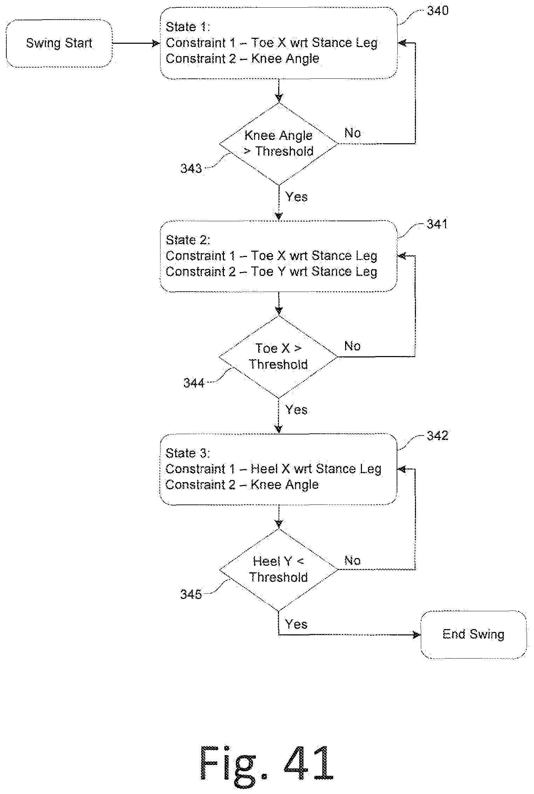

Figure 41 is a flow chart for a simple finite state machine employed with the exoskeleton system of the invention; and
Figure 42 is a modified, more complex, version of a flow chart for use in connection with the invention.

### DETAILED DESCRIPTION OF THE INVENTION

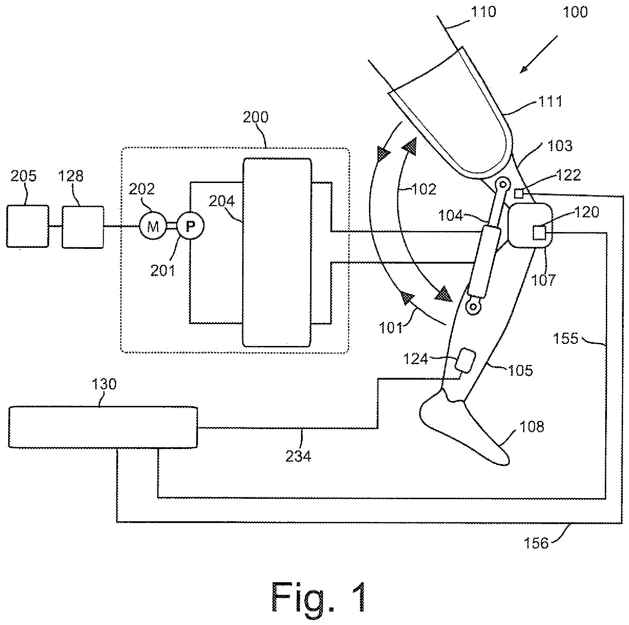

With initial reference to Figure 1, a semi-actuated prosthetic knee 100 constructed in accordance with a first embodiment of the invention is configurable to be coupled to an above-knee amputee's remaining lower limb 110 through a socket 111. Semi-actuated prosthetic knee 100, among other components, comprises a thigh link 103 coupled to a knee mechanism 107 and a shank link 105 coupled to an artificial foot 108. Knee mechanism 107 is configured to allow flexion and extension movements of thigh link 103 and a shank link 105 relative to each other along flexion direction 101 and extension direction 102. A hydraulic torque generator 104 is configured to generate torque between thigh link 103 and shank link 105.

Semi-actuated prosthetic knee 100 further includes a hydraulic power unit indicated at 200 coupled to hydraulic torque generator 104. Hydraulic power unit 200, among other components, includes a hydraulic valve circuit 204, which is hydraulically coupled to torque generator 104. Hydraulic power unit 200 further includes a hydraulic pump 201 mechanically coupled to an electric motor 202 and hydraulically coupled to hydraulic valve circuit 204.

Semi-actuated prosthetic knee 100 further includes an electric power source 205 capable of providing electric power to electric motor 202 and other components of semi-actuated prosthetic knee 100. A motor controller 128 (sometimes referred to as an amplifier) converts the output of electric power source 205 to an appropriate voltage or current for electric motor 202. Semi-actuated prosthetic knee 100 further includes a signal processor 130 that among other tasks controls electric motor 202 and implements a controller that includes a set of states. Semi-actuated prosthetic knee 100 additionally includes a stance sensor 124 producing stance signal 234. Stance signal 234, among other information, includes information identifying which part of artificial foot 108 is in contact with the ground.

In operation when semi-actuated prosthetic knee 100 is in its actuated mode, semi-actuated prosthetic knee 100 is configured such that it transfers electric power from electric power source 205 to electric motor 202, powering electric motor 202 and hydraulic pump 201. In this actuated mode, hydraulic valve circuit 204 is configured such that hydraulic pump 201 hydraulically couples to torque generator 104. This hydraulic coupling between hydraulic pump 201 and torque generator 104 allows signal processor 130 to control torque generator 104. The ability to inject power to torque generator 104 allows one to control the motion of knee mechanism 107 or impose desirable torque onto knee mechanism 107 during various phases of the walking cycle.

When semi-actuated prosthetic knee 100 is in an un-actuated mode, hydraulic power unit 200 is configured such that no electric power from electric power source 205 is transferred to electric motor 202. In this un-actuated mode hydraulic valve circuit 204 modulates the resistance of the fluid flow in torque generator 104. The ability to modulate the resistance of fluid flow in torque generator 104 allows one to control the resistance of knee mechanism 107 to forces and torques during various phases of the walking cycle with reduced use of electric power since electric motor 202 is not consuming any electric power in this un-actuated mode.

Examples of hydraulic torque generators 104 include, without limitation, linear hydraulic piston-cylinders, rotary hydraulic actuators, rack-and-pinion-type rotary actuators and rotary hydraulic vane type actuators where pressurized hydraulic fluid, by pushing against moving surfaces, generate force or torque.

Examples of electric power source 205 include, without limitation, batteries, Nickel-Metal Hydride (NiMH) batteries, Lithium batteries, Alkaline batteries, rechargeable Alkaline batteries, Lithium-ion batteries, and Lithium ion polymer batteries.

Examples of electric motor 202 include, without limitation, electric motors, including, without limitation, AC (alternating current) motors, brush-type DC (direct current) motors, brushless DC motors, electronically commutated motors (ECMs), stepping motors, and combinations thereof.

Examples of hydraulic pump 201 include, without limitation, gear pumps, gerotor pumps, rotary vane pumps, screw pumps, bent axis pumps, axial piston pumps swashplate pumps, radial piston pumps, and peristaltic pumps.

Examples of stance sensor 124 include, without limitation, force sensors, strain gage force sensors, piezoelectric force sensors, force sensing resistors, load cells, deflection-based positioning sensors, encoders, potentiometers, pressure sensors in a trapped hydraulic fluid, and combinations thereof.

Examples of knee mechanism 107 include, without limitation, rotary pivots, four-bar linkages, sliding joints, rolling element joints, and combinations thereof.

Signal processor 130 comprises an element or combination of elements selected from the group consisting of analog devices; analog computation modules; digital devices including, without limitation, small-, medium-, and large-scale integrated circuits, application specific integrated circuits, programmable gate arrays, programmable logic arrays; electromechanical relays, solid state switches, MOSFET switches and digital computation modules including, without limitation, microcomputers, microprocessors, microcontrollers, and programmable logic controllers. In operation signal processor 130 collects information from various sensors and after some computation commands what various components of hydraulic circuit should do.

In some embodiments of the invention, as shown in Figure 1, semi-actuated prosthetic knee 100 further comprises a knee angle sensor 120 which generates a knee angle signal indicated at 155 representing the angle between thigh link 103 and shank link 105. Knee angle sensor 120 comprises an element or combination of elements selected from a the group consisting of an encoder, digital encoder, magnetic encoder, optical encoder, potentiometer, LVDT, and resolver.

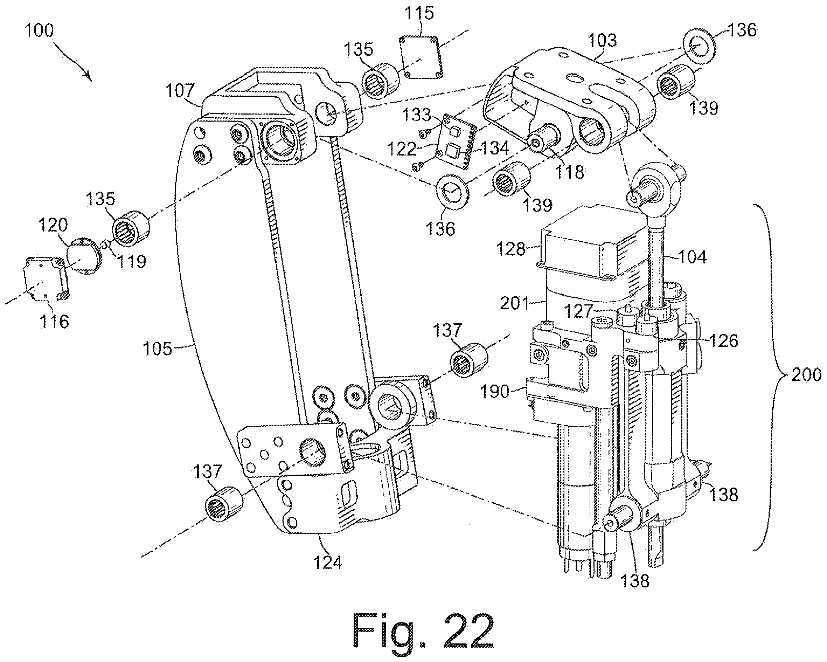

In some embodiments, as shown in Figure 1, semi-actuated prosthetic knee 100 further comprises a thigh angle sensor 122, which generates a thigh angle signal indicated at 156 representing the absolute angle of thigh link 103. Thigh angle sensor 122 comprises an element or combination of elements selected from a the group consisting of, accelerometers, gyroscopes, inclinometers, encoders, potentiometers and combinations thereof. Figure 22 represents an embodiment of the invention where thigh angle sensor 122 fixed to thigh link 103 comprises an accelerometer 133 and a gyroscope 134.

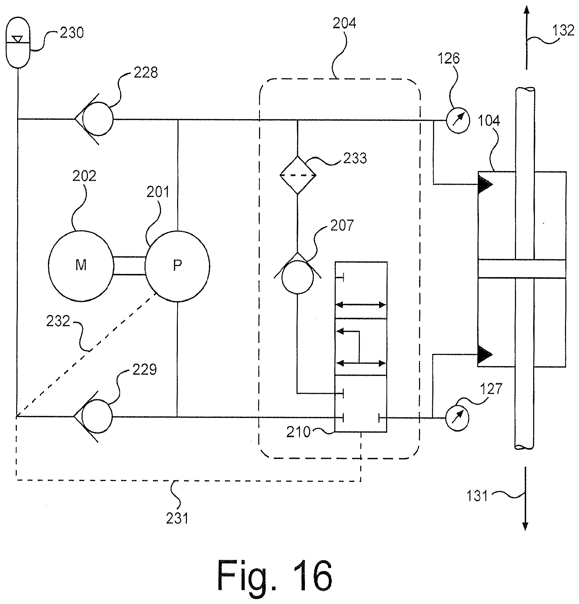

In some embodiments of the invention semi-actuated prosthetic knee 100 further comprises a torque sensor or a force sensor (as detailed below) representing the torque or force of torque generator 104. In some embodiments of the invention a force sensor is installed on the piston of linear torque generator 104. In some embodiments of the invention, the force sensor for semi-actuated prosthetic knee 100 comprises two pressure sensors 126 and 127 measuring the fluid pressure in both sides of torque generator 104, as depicted in Figure 16. The measurements from two pressure sensors 126 and 127 also represent the force in torque generator torque generator 104.

In some embodiments as shown in Figure 1, stance sensor 124 comprises a force-torque sensor installed on shank link 105 measuring the force and the moment in the sagittal plane.

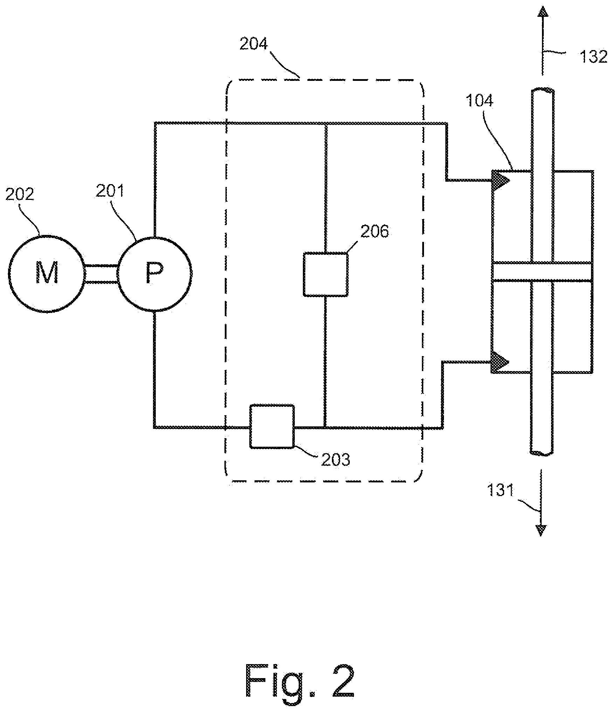

In some embodiments, as shown in Figure 2, hydraulic valve circuit 204 comprises a first controllable valve 206 capable of allowing the hydraulic flow in two directions and a pump valve 203 serially connected to each other. Hydraulic pump 201 is coupled to two end ports of this serially-connected chain of first controllable valve 206 and pump valve 203. Torque generator 104 is coupled to two ports of first controllable valve 206. In some cases, when semi-actuated prosthetic knee 100 operates in its actuated mode, first controllable valve 206 is closed. This allows the entire hydraulic pump output flow to travel to torque generator 104. This further allows signal processor 130 to control torque generator 104 by controlling electric motor 202. The ability to inject power to torque generator 104, in the actuated mode, allows one to control the motion of knee mechanism 107 or impose desirable torque onto knee mechanism 107.

When semi-actuated prosthetic knee 100 operates in its un-actuated mode, pump valve 203 is either closed or partially closed. When pump valve 203 is fully closed, no flow passes through hydraulic pump 201. Through the use of signal processor 130, one can adjust the opening of first controllable valve 206 to modulate and adjust properly the resistance of fluid flow in torque generator 104. When pump valve 203 is partially closed, one can only modulate the resistance of fluid flow in torque generator 104 from zero to the combined flow resistance of pump valve 203 and hydraulic pump 201. The ability to modulate the resistance of fluid flow in torque generator 104 allows one to control the resistance of knee mechanism 107 to forces and torques with reduced use of electric power since electric motor 202 is not consuming any electric power in this un-actuated mode.

When semi-actuated prosthetic knee 100 operates in a power regenerative mode, pump valve 203 is not closed, allowing at least a portion of the hydraulic flow from torque generator 104 to turn hydraulic pump 201 while motor controller 128 applies a non-zero current onto electric motor 202 to resist the hydraulic flow in hydraulic pump 201.

For better clarification of the embodiments of hydraulic valve circuit 204, the flexion and extension will be defined as follows. The flexion of prosthetic knee 100 takes place when the piston of torque generator 104 moves in direction of arrow 131 depicted in Figure 2. Extension of prosthetic knee 100 takes place when the piston of torque generator 104 moves in direction of arrow 132 depicted in Figure 2.

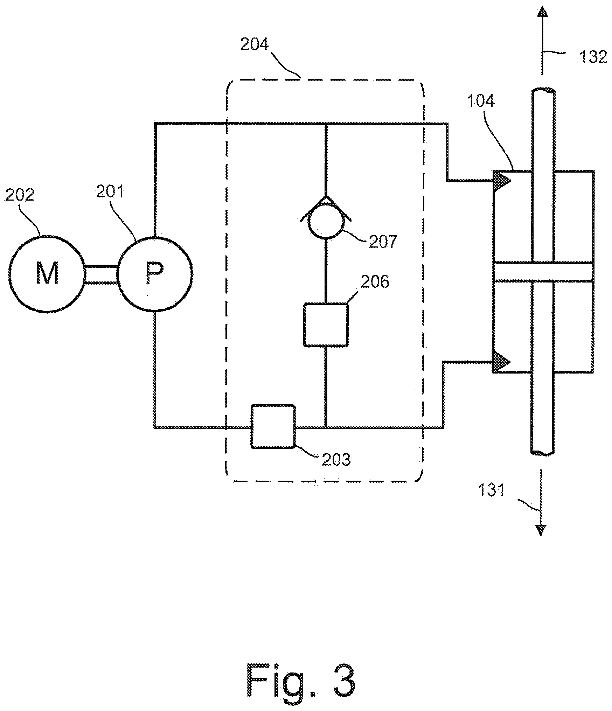

In some embodiments, as shown in Figure 3, hydraulic valve circuit 204, among other components, further comprises a first check valve 207 installed in series with first controllable valve 206. The operation of this embodiment is similar to the operation of the embodiment shown in Figure 2, except that first hydraulic controllable valve 206 modulates the resistance of the fluid flow in torque generator 104 in one direction only. In comparison with the embodiment of Figure 2, this embodiment constrains the range of resistance of fluid flow in torque generator 104 in flexion direction to always be more than the flow resistance that hydraulic pump 201 creates. It further allows free extension of torque generator 104 if first controllable valve 206 is open without compromising the ability to inject power in the extension direction of torque generator 104. Similar to the embodiment of Figure 2, when semi-actuated prosthetic knee 100 operates in its actuated mode, first controllable valve 206 is closed. This allows signal processor 130 to control torque generator 104 by controlling electric motor 202. The ability to inject power to torque generator 104, in the actuated mode, allows one to control the motion of knee mechanism 107 or impose desirable torque onto knee mechanism 107.

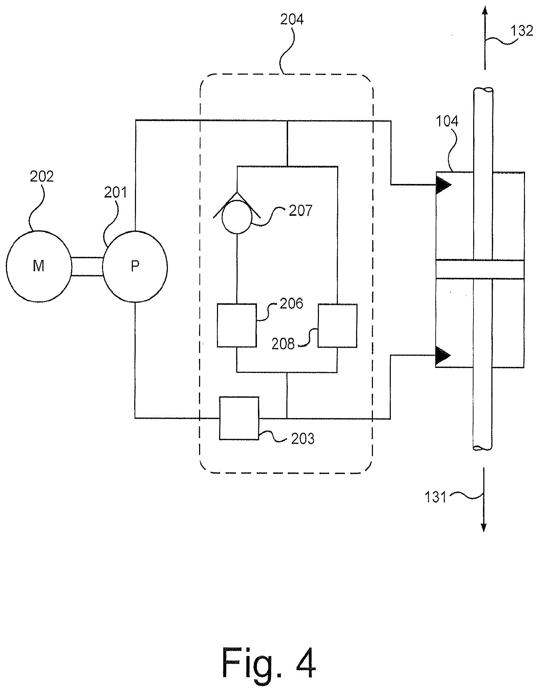

In some embodiments, as shown in Figure 4, hydraulic valve circuit 204, among other components, further comprises a second controllable valve 208 installed in parallel with serially-installed first controllable valve 206 and first check valve 207. Through the use of signal processor 130, one can adjust the opening of first controllable valve 206 and second controllable valve 208 to modulate and adjust properly the resistance of fluid flow in torque generator 104. The operation of this embodiment is similar to the operation of the embodiment shown in Figure 3, except that this embodiment does not constrain the range of resistance of fluid flow in flexion direction in torque generator 104. When semi-actuated prosthetic knee 100 operates in its actuated mode, first controllable valve 206 and second controllable valve 208 are closed. This allows signal processor 130 to control torque generator 104 by controlling electric motor 202. The ability to inject power to torque generator 104, in the actuated mode, allows one to control the motion of knee mechanism 107 or impose desirable torque onto knee mechanism 107.

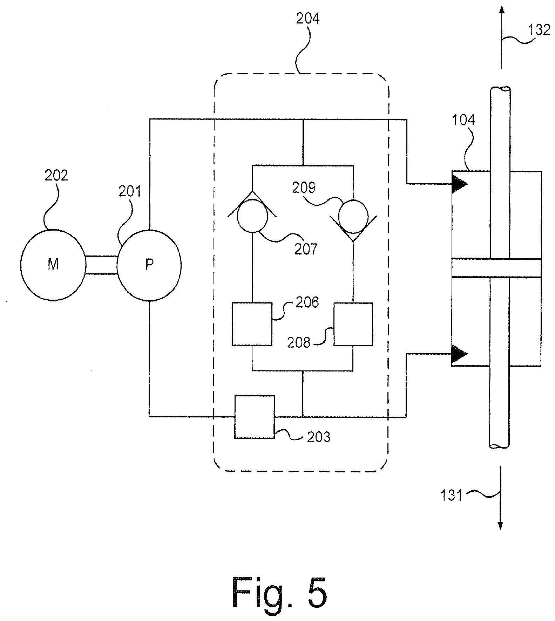

In some embodiments, as shown in Figure 5, hydraulic valve circuit 204, includes a second check valve 209 and second controllable valve 208 installed in series relative to each other and installed in parallel with serially installed first controllable valve 206 and first check valve 207. The operation of this embodiment is similar to the operation of the embodiment shown in Figure 4 except it allows free flexion of torque generator 104 if second controllable valve 208 is open without compromising the ability to inject power in the flexion direction of torque generator 104. Similar to the embodiment of Figure 4, when hydraulic valve circuit 204 of Figure 5 operates in its actuated mode, first controllable valve 206 and second controllable valve 208 are closed and that allows one to control the motion of knee mechanism 107 or impose desirable torque onto knee mechanism 107.

Both first controllable valve 206 and second controllable valve 208 comprise any valve or combination of valves that allow for variation or adjustment of their openings either electronically or manually. Examples of first controllable valve 206 and second controllable valve 208 include, without limitation, a flow control valve, a pressure control valve, actuated needle valves, solenoid valves and an on-off valve.

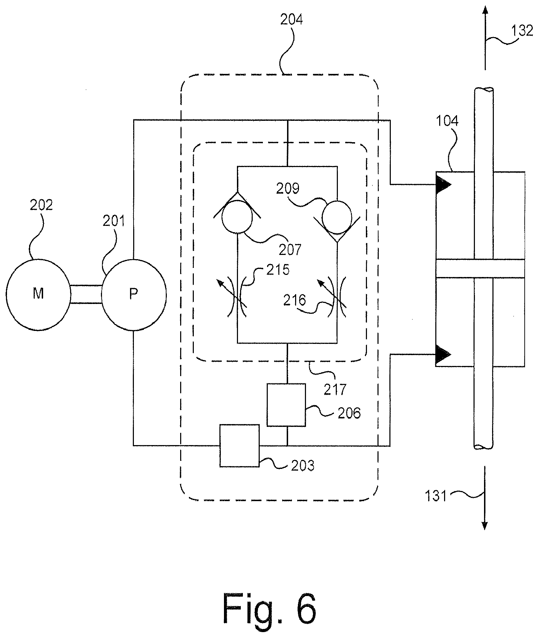

Figure 6 shows another embodiment of hydraulic valve circuit 204. The embodiment of hydraulic valve circuit 204 of Figure 6 is the same as embodiment of Figure 3 except first check valve 207 in Figure 3 is replaced by parallel path circuit 217. Parallel path circuit 217 comprises a first check valve 207 and a first adjustable restrictor valve 215 installed in series relative to each other and installed in parallel with serially installed second check valve 209 and a second adjustable restrictor valve 216.

In operation, when semi-actuated prosthetic knee 100 operates in its actuated mode, first controllable valve 206 is closed. This allows the entire hydraulic pump output flow to travel to torque generator 104. This further allows signal processor 130 to control torque generator 104 by controlling electric motor 202. The ability to inject power to torque generator 104, in actuated mode, allows one to control the motion of knee mechanism 107 or impose desirable torque onto knee mechanism 107. When semi-actuated prosthetic knee 100 operates in its un-actuated mode, pump valve 203 is closed so that no flow passes through hydraulic pump 201. Through the use of signal processor 130, one can adjust the opening of first controllable valve 206 to modulate the resistance of fluid flow in torque generator 104. Adjustable restrictor valve 215 is adjusted to provide resistance to fluid flow in the extension direction of torque generator 104. Adjustable restrictor valve 216 is adjusted to provide resistance to fluid flow in the flexion direction of torque generator 104. The ability to modulate the resistance of fluid flow in torque generator 104 allows one to control the resistance of knee mechanism 107 to forces and torques, with reduces use of electric power since electric motor 202 is not consuming any electric power in this un-actuated mode.

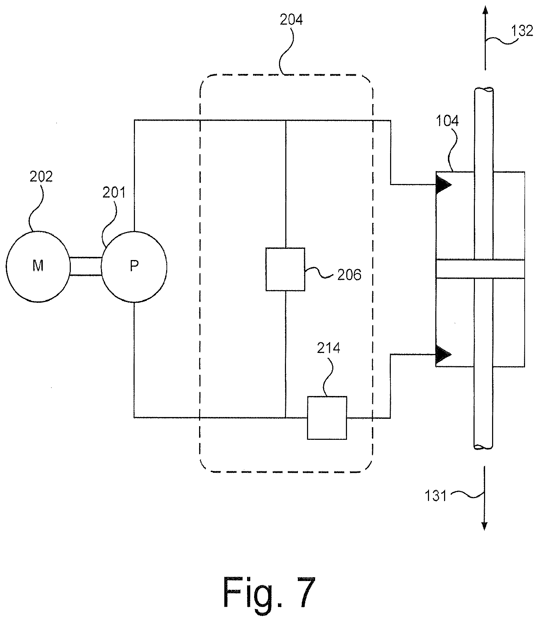

In some embodiments, as shown in Figure 7, hydraulic valve circuit 204 comprises a first controllable valve 206 capable of controlling the hydraulic flow in two directions and an actuator valve 214 serially connected to each other. In this embodiment, torque generator 104 is coupled to two free ports of this serially connected first controllable valve 206 and said actuator valve 214. Hydraulic pump 201 is coupled to two ports of first controllable valve 206.

In operation, when semi-actuated prosthetic knee 100 operates in its actuated mode, first controllable valve 206 is closed. This allows the entire hydraulic pump output flow to travel to torque generator 104. This further allows signal processor 130 to control torque generator 104 by controlling electric motor 202. The ability to inject power to torque generator 104, in actuated mode, allows one to control the motion of knee mechanism 107 or impose desirable torque onto knee mechanism 107. When semi-actuated prosthetic knee 100 operates in its un-actuated mode, through the use of signal processor 130, one can adjust the opening of actuator valve 214 to modulate the resistance of fluid flow in torque generator 104. The ability to modulate the resistance of fluid flow in torque generator 104 allows one to control the resistance of knee mechanism 107 to forces and torques with reduced use of electric power since electric motor 202 is not consuming any electric power in this un-actuated mode.

When semi-actuated prosthetic knee 100 operates in a power regenerative mode, actuator valve 214 is not closed, allowing at least a portion of the hydraulic flow from torque generator 104 to turn hydraulic pump 201 while motor controller 128 applies a non-zero current onto electric motor 202 to resist the hydraulic flow in hydraulic pump 201.

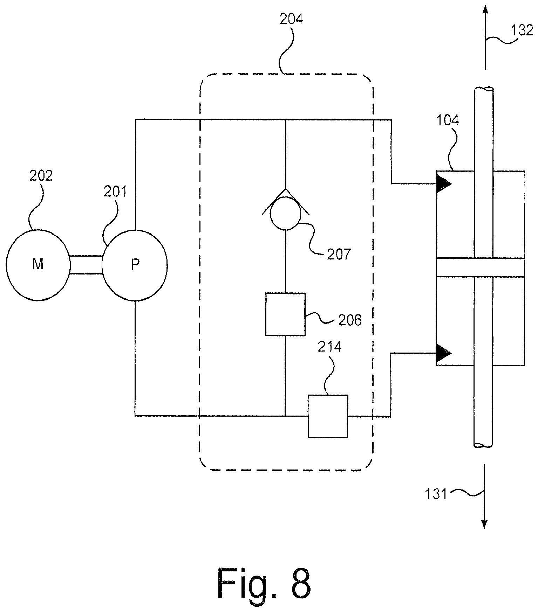

In some embodiments, as shown in Figure 8, hydraulic valve circuit 204, among other components, further comprises a first check valve 207 installed in series with first controllable valve 206 allowing the hydraulic flow in one direction only. In comparison with the embodiment of Figure 7, this embodiment constrains the resistance of fluid flow in torque generator 104 in the flexion direction to always be more than the flow resistance that hydraulic pump 201 creates. It further allows free extension of torque generator 104 if first controllable valve 206 is open without compromising the ability to inject power in the extension direction of torque generator 104. When semi-actuated prosthetic knee 100 operates in its actuated mode, first controllable valve 206 is closed. This allows one to control the motion of knee mechanism 107 or impose desirable torque onto knee mechanism 107.

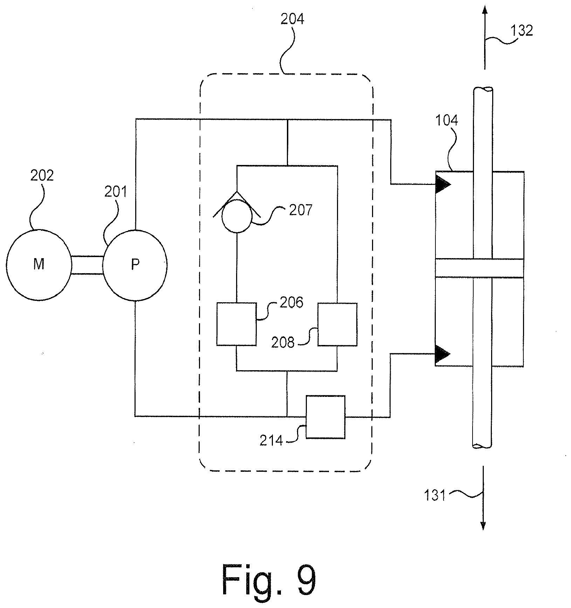

In some embodiments, as shown in Figure 9, hydraulic valve circuit 204, among other components, further comprises a second controllable valve 208 installed in parallel with serially-installed first controllable valve 206 and first check valve 207. The operation of this embodiment is similar to the operation of the embodiment shown in Figure 8 except this embodiment does not constrain the resistance of fluid flow in torque generator 104 in the flexion direction to always be more than the flow resistance that hydraulic pump 201 creates. In operation, when hydraulic valve circuit 204 of Figure 9 operates in its actuated mode, first and second controllable valves 206 and 208 are closed. This allows the entire hydraulic pump output flow to travel to torque generator 104. This further allows signal processor 130 to control torque generator 104 by controlling electric motor 202. The ability to inject power to torque generator 104, in actuated mode, allows one to control the motion of knee mechanism 107 or impose desirable torque onto knee mechanism 107.

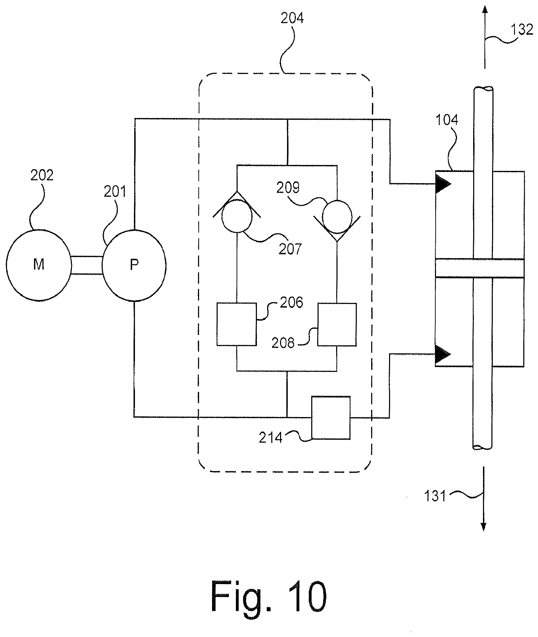

In some embodiments, as shown in Figure 10, hydraulic valve circuit 204 comprises a second check valve 209 and second controllable valve 208 installed in series relative to each other and installed in parallel with serially installed first controllable valve 206 and first check valve 207. The operation of this embodiment is similar to the operation of the embodiment shown in Figure 9 except it allows free flexion of torque generator 104 if second controllable valve 208 is open without compromising the ability to inject power in the flexion direction of torque generator 104. When semi-actuated prosthetic knee 100 operates in its actuated mode, first and second controllable valves 206 and 208 are closed. This allows one to control the motion of knee mechanism 107 or impose desirable torque onto knee mechanism 107.

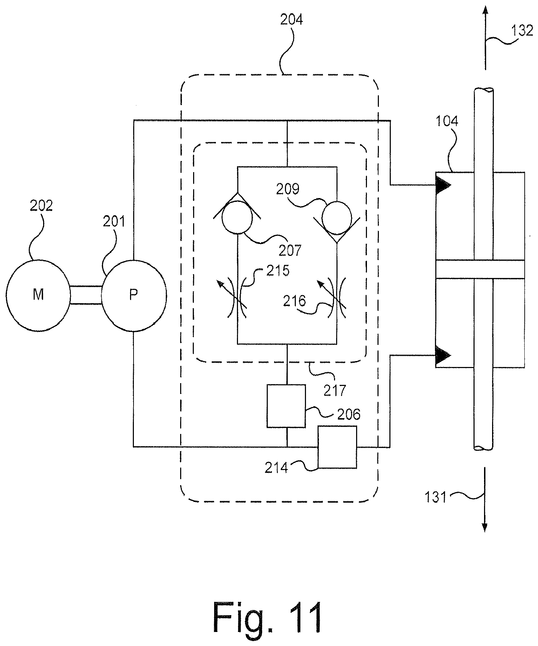

Figure 11 shows another embodiment of hydraulic valve circuit 204. The embodiment of hydraulic valve circuit 204 of Figure 11 is the same as embodiment of Figure 8 except check valve 207 in Figure 8 is replaced by parallel path circuit 217. Parallel path circuit 217 comprises a first check valve 207 and first adjustable restrictor valve 215 installed in series relative to each other and installed in parallel with serially installed second check valve 209 and second adjustable restrictor valve 216.

In operation, when semi-actuated prosthetic knee 100 operates in its actuated mode, first controllable valve 206 is closed. This allows the entire hydraulic pump output flow to travel to torque generator 104. This further allows signal processor 130 to control torque generator 104 by controlling electric motor 202. The ability to inject power to torque generator 104, in actuated mode, allows one to control the motion of knee mechanism 107 or impose desirable torque onto knee mechanism 107. When semi-actuated prosthetic knee 100 operates in its un-actuated mode, one can adjust the opening of actuator valve 214 to modulate the resistance of fluid flow in torque generator 104. First adjustable restrictor valve 215 is adjusted to provide resistance to fluid flow in the extension direction of torque generator 104. Second adjustable restrictor valve 216 is adjusted to provide resistance to fluid flow in the flexion direction of torque generator 104. The ability to modulate the resistance of fluid flow in torque generator 104 allows one to control the resistance of knee mechanism 107 to forces and torques with reduced use of electric power since electric motor 202 is not consuming any electric power in this un-actuated mode.

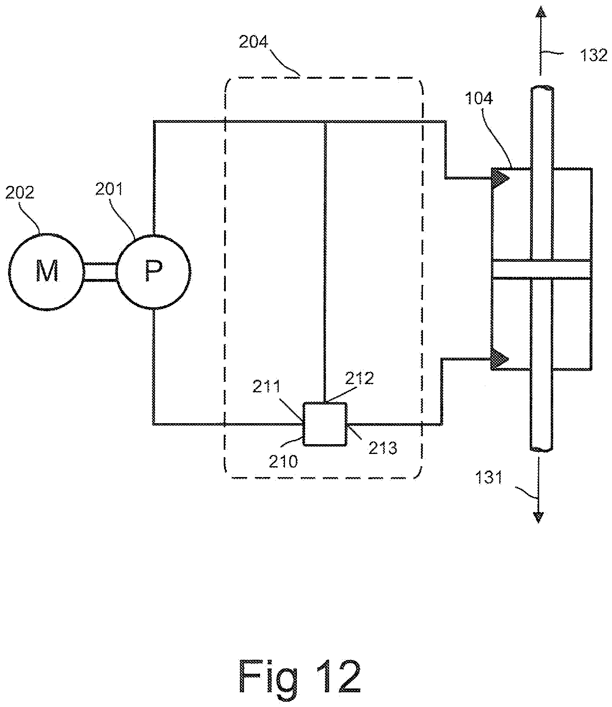

In some embodiments, as shown in Figure 12, hydraulic valve circuit 204 comprises a three-way valve 210 capable of controlling the hydraulic flow. In operation, when semi-actuated prosthetic knee 100 operates in its actuated mode, three-way valve connects port 211 to port 213 and blocks port 212. This allows for fluid flow between hydraulic pump 201 and torque generator 104 such that the entire hydraulic pump output flow travels to torque generator 104. This further allows signal processor 130 to control torque generator 104 by controlling electric motor 202. The ability to inject power to torque generator 104, in this actuated mode, allows one to control the motion of knee mechanism 107 or impose desirable torque onto knee mechanism 107. When semi-actuated prosthetic knee 100 operates in an un-actuated mode, three-way valve 210 connects port 212 to port 213. Through the use of signal processor 130, one can adjust the opening of port 213 to modulate the resistance of fluid flow in torque generator 104. The ability to modulate the resistance of fluid flow in torque generator 104 allows one to control the resistance of knee mechanism 107 to forces and torques with reduced use of electric power since electric motor 202 is not consuming any electric power in this un-actuated mode. When semi-actuated prosthetic knee 100 operates in a power regenerative mode, three-way valve 210 connects port 211 to port 213 allowing at least a portion of the hydraulic flow from torque generator 104 to turn hydraulic pump 201 while motor controller 128 applies a non-zero current onto electric motor 202 to resist the hydraulic flow in hydraulic pump 201.

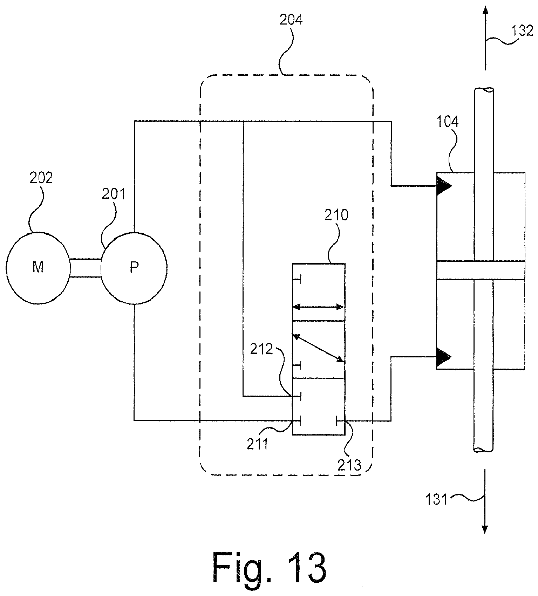

Figure 13 shows a realization of the embodiment of Figure 12. More specifically, Figure 13 shows a three-way valve 210 that has at least three positions. When three-way valve 210 is in its first position, three-way valve connects port 211 to port 213 and blocks port 212. This allows semi-actuated prosthetic knee 100 to operate in actuated mode. When three-way valve 210 is in its second position, it connects port 212 to port 213 and blocks port 211. Through the use of signal processor 130, one can adjust the opening of port 212, port 213 or both port 212 and 213 to modulate and adjust properly the resistance of fluid flow in torque generator 104. When three-way valve 210 is in its third position (shown in Figure 13), none of the ports are connected to each other.

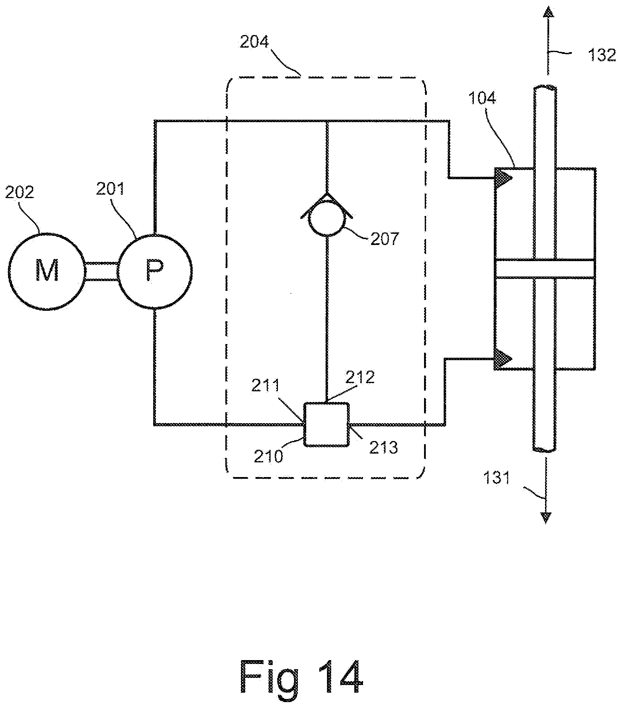

Figure 14 shows another embodiment of the embodiment of Figure 12 where hydraulic valve circuit 204 further comprises a first check valve 207 coupled to port 212. In comparison with the embodiment of Figure 12, this embodiment constrains the range of resistance of fluid flow in torque generator 104 in flexion direction to always be more than the flow resistance that hydraulic pump 201 creates. It further allows free extension of torque generator 104 if all ports 211, 212 are 213 are connected to each other without compromising the ability to inject power in the extension direction of torque generator 104. When semi-actuated prosthetic knee 100 operates in its actuated mode, three-way valve 210 connects port 211 to port 213 and blocks port 212. This allows for fluid flow between hydraulic pump 201 and torque generator 104 such that the entire hydraulic pump output flow travels to torque generator 104. This further allows signal processor 130 to control the motion of knee mechanism 107 or impose desirable torque onto knee mechanism 107 by controlling electric motor 202.

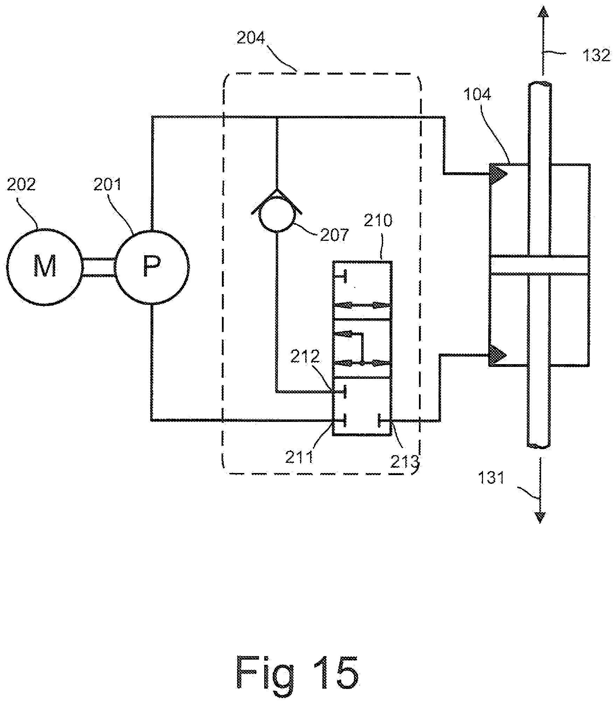

Figure 15 shows a realization of the embodiment of Figure 14. Figure 15 shows a three valve 210 that has at least three positions. When three-way valve 210 is in its first position (actuated mode), three-way valve 210 connects port 211 to port 213 and blocks port 212. When three-way valve 210 is in its second position, all ports are connected to each other. Through the use of signal processor 130, one can adjust the opening of port 212, port 213 or both port 212 and 213 to properly modulate and adjust the resistance of fluid flow in torque generator 104. When three-way valve 210 is in its third position (shown in Figure 15), none of the ports are connected to each other.

Figure 16 shows the same embodiment of Figure 15 with a few added features. A reservoir 230 ensures sufficient oil is in the system in the presence of any leakage or thermal expansion. Two check valves 228 and 229 ensure hydraulic fluid is not pushed back to reservoir 230. Two hydraulic fluid paths 231 and 232 ensure any leakage from the three-way valve 210 and hydraulic pump 201 are fed back to reservoir 230. Pressure sensors 126 and 127 measure the hydraulic fluid pressure in first and second chambers of torque generator 104. A filter 233 collects any contaminants in the fluid.

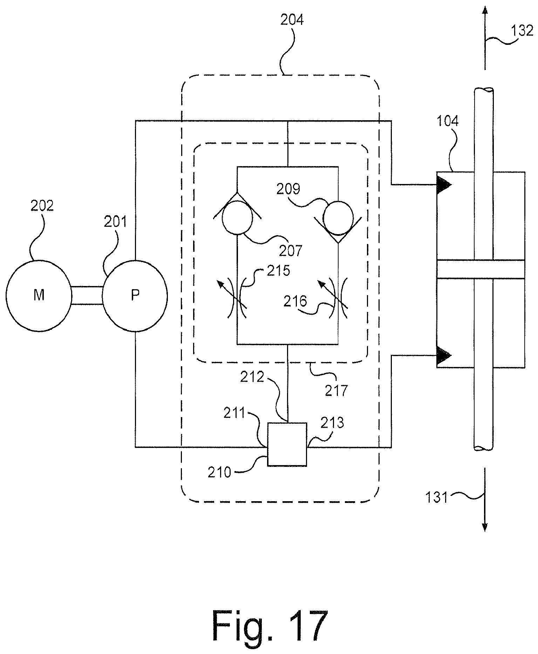

Figure 17 shows another embodiment of Figure 12 wherein hydraulic valve circuit 204 further comprises a parallel path circuit 217 coupled to port 212. In operation, when semi-actuated prosthetic knee 100 operates in its actuated mode, three-way valve 210 connects port 211 to port 213 and blocks port 212. This allows for fluid flow between hydraulic pump 201 and torque generator 104 such that the entire said hydraulic pump output flow travels to torque generator 104. This further allows signal processor 130 to control torque generator 104 by controlling electric motor 202. The ability to inject power to torque generator 104 in this actuated mode allows one to control the motion of knee mechanism 107 or impose desirable torque onto knee mechanism 107. When semi-actuated prosthetic knee 100 operates in its un-actuated mode, three-way valve 210 connects port 212 to port 213 and blocks port 211. Through the use of signal processor 130, one can adjust the opening of port 213 or port 212 to modulate the resistance of fluid flow in torque generator 104. First adjustable restrictor valve 215 is adjusted to provide resistance to fluid flow in the extension direction of torque generator 104. Second adjustable restrictor valve 216 is adjusted to provide resistance to fluid flow in the flexion direction of torque generator 104. The ability to modulate the resistance of fluid flow in torque generator 104 allows one to control the resistance of knee mechanism 107 to forces and torques with reduced use of electric power since electric motor 202 is not consuming any electric power in this un-actuated mode.

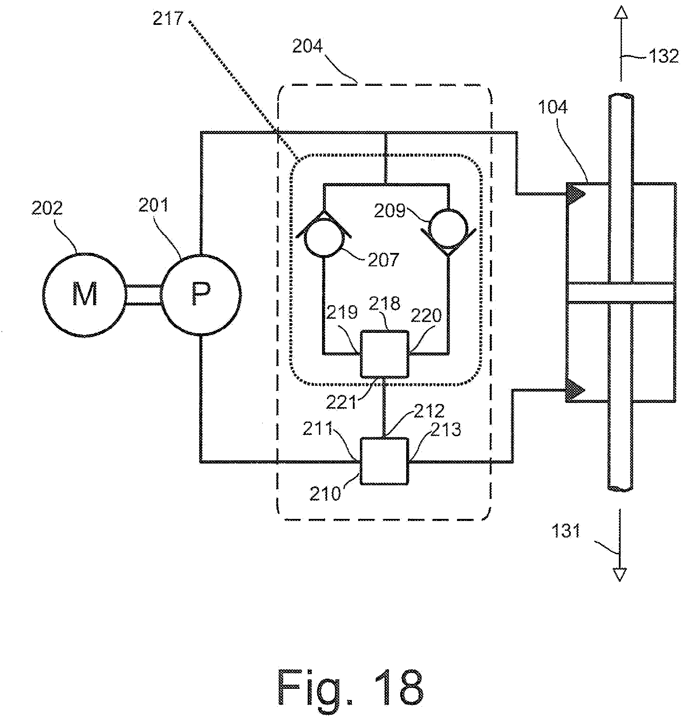

Figure 18 shows another embodiment of hydraulic valve circuit 204. The embodiment of Figure 18 is the same as the embodiment of Figure 17 except adjustable restrictor valves 215 and 216 are replaced by a second three-way valve 218. In operation when semi-actuated prosthetic knee 100 operates in an actuated mode, three-way valve 210 connects port 211 to port 213 and blocks port 212. This allows for fluid flow between hydraulic pump 201 and torque generator 104 such that the entire hydraulic pump output flow travels to torque generator 104. This further allows signal processor 130 to control torque generator 104 by controlling electric motor 202. When semi-actuated prosthetic knee 100 operates in an un-actuated mode, first three-way valve 210 connects port 212 to port 213. Second three-way valve 218 modulates the resistance to hydraulic flow between a port 219 and a port 221 when torque generator 104 moves in the extension direction and modulates the resistance to hydraulic flow between a port 220 and port 221 when torque generator 104 moves in the flexion direction. This embodiment allows free extension of torque generator 104 without compromising the ability to inject power in the extension direction of torque generator 104 if port 219 and port 221 are connected and port 220 is blocked and if ports 211, 212 and 213 are connected to each other. This embodiment further allows free flexion of torque generator 104 without compromising the ability to inject power in the flexion direction of torque generator 104 if port 220 and port 221 are connected and port 219 is blocked and if ports 211, 212 and 213 are connected to each other.

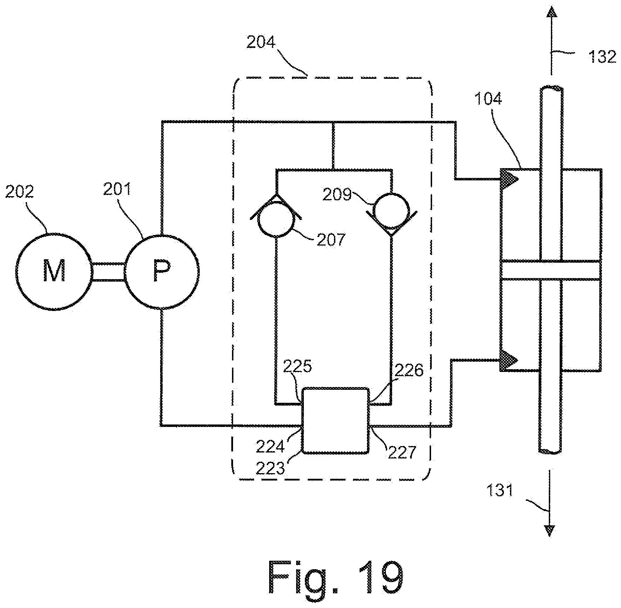

Figure 19 shows another embodiment of hydraulic valve circuit 204. The embodiment of Figure 19 is the same as the embodiment of Figure 18 except two three-way valves 210 and 218 are replaced by a four way valve 223. In operation when semi-actuated prosthetic knee 100 operates in an actuated mode, four-way valve 223 connects a port 224 to a port 227 and blocks ports 225 and 226. This allows for fluid flow between hydraulic pump 201 and torque generator 104 such that the entire said hydraulic pump output flow travels to torque generator 104. This further allows signal processor 130 to control torque generator 104 by controlling electric motor 202. When semi-actuated prosthetic knee 100 operates in an un-actuated mode, four-way valve 223 modulates the resistance to hydraulic flow between port 225 and port 227 when torque generator 104 moves in the extension direction and modulates the resistance to hydraulic flow between port 226 and port 227 when torque generator 104 moves in the flexion direction. This embodiment allows free extension of torque generator 104 without compromising the ability to inject power in the extension direction of torque generator 104 if ports 224, 225, and 227 are connected and port 226 is blocked. This embodiment further allows free flexion of torque generator 104 without compromising the ability to inject power in the flexion direction of torque generator 104 if ports 224, 226, and 227 are connected and port 225 is blocked.

As can be seen from Figures 1 through 19, hydraulic power unit 200 comprises two paths that connect to torque generator 104: one through hydraulic pump 201 and the second through a hydraulic valve circuit 204. In the actuated mode, hydraulic pump 201 hydraulically couples to torque generator 104. In un-actuated mode, the flow to torque generator 104 is modulated by at least one valve.

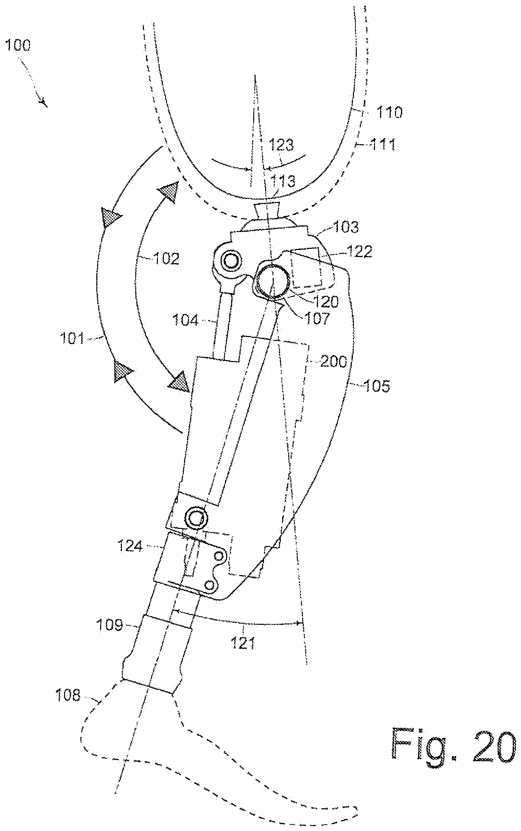

Figure 20 represents the schematic of one embodiment of semi-actuated prosthetic knee 100. As previously noted, semi-actuated prosthetic knee 100, among other components, comprises a thigh link 103, a shank link 105, and a knee mechanism 107, coupled by torque generator 104. Knee mechanism 107 is configured to allow movement of thigh link 103 relative to shank link 105 along flexion direction 101 and extension direction 102. Semi-actuated prosthetic knee 100 is configurable to be coupled to an above-knee amputee's remaining lower limb 110 through a socket 111. More specifically, socket 111 is coupled to thigh link 103 with a pyramid adapter 113 or similar adapter known in the art. An ankle pylon 109 connects shank link 105 to artificial foot 108 through stance sensor 124. Knee angle sensor 120 measures an angle 121 between thigh link 103 and shank link 105. Thigh angle sensor 122 located on thigh link 103 measures an absolute angle 123 of thigh link 103. The profile of hydraulic power unit 200 is shown in Figure 20.

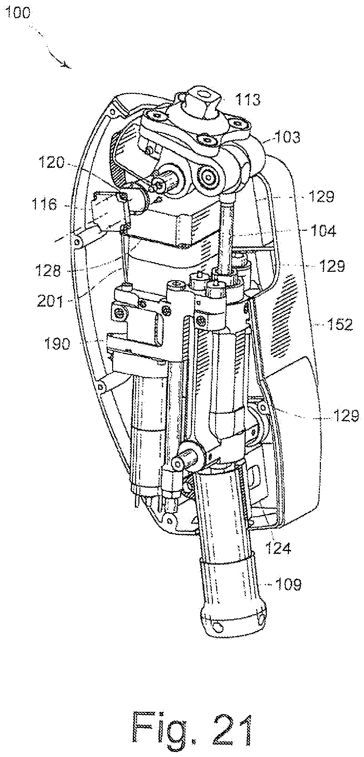

Figures 21 and 22 represent a cutaway perspective drawing and exploded view of the semi-actuated prosthetic knee 100 presented in Figure 20. In the embodiment of Figures 21 and 22, pyramid adapter 113 connects to thigh link 103. Thigh angle sensor 122 fixed to thigh link 103 comprises an accelerometer 133 and a gyroscope 134. A shaft 118 extending from thigh link 103 is stationary with respect to thigh link 103. Knee angle sensor 120 is in the form of a magnetic encoder fixed to an encoder housing 116 and stationary with respect to shank link 105. Magnetic encoder 120 measures the angle of a magnet 119 embedded in shaft 118. Shaft 118 is secured to thigh link 103 and turns inside needle bearings 135. Thrust bushings 136 provide axial support between thigh link 103 and knee mechanism 107. A bearing cover 115 protects needle bearing 135. Hydraulic power unit 200 comprises, among other elements, motor controller 128, hydraulic pump 201, a hydraulic manifold 190, torque generator 104 and pressure sensors 126 and 127. Power unit 200 pivots with respect to shank link 105 on needle bearings 137. Thrust bushings 138 provide axial support between power unit 200 and shank link 105. Torque generator 104 couples to thigh link 103 through needle bearings 139 to complete the linkage between thigh link 103, shank link 105, and torque generator 104. Stance sensor 124 connects shank link 105 to ankle pylon 109. Batteries 129 are used to provide electric power for the prosthetic knee 100.

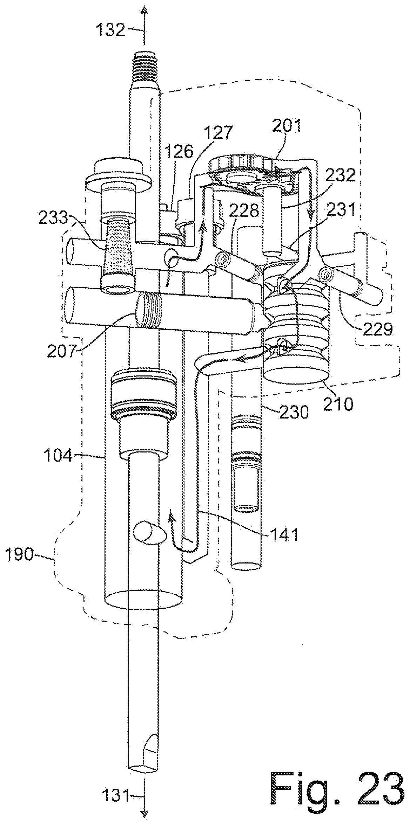

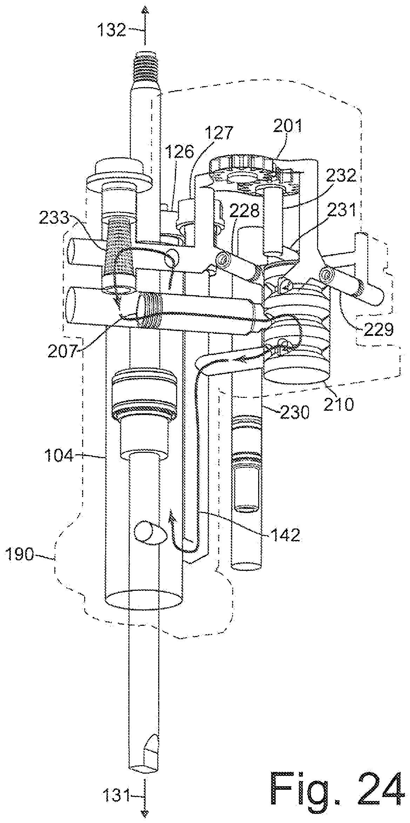

Figure 23 shows a perspective drawing of the hydraulic valve circuit shown in Figure 16. An arrow 141 represents the path of hydraulic flow during an actuated mode in extension direction represented by arrow 132. Three-way valve 210 incorporates three ports 211, 212, and 213 (depicted in Figure 16) that connect to hydraulic pump 201, check valve 207 and torque generator 104, respectively. Check valves 228 and 229 prevent the fluid flow back to reservoir 230. Hydraulic fluid paths 231 and 232 define passages from hydraulic pump 201 and three-way hydraulic valve 210 to reservoir 230. Figure 24 also shows a perspective drawing of the hydraulic valve circuit of Figure 16, where an arrow 142 shows the path of the hydraulic flow during un-actuated mode in extension direction.

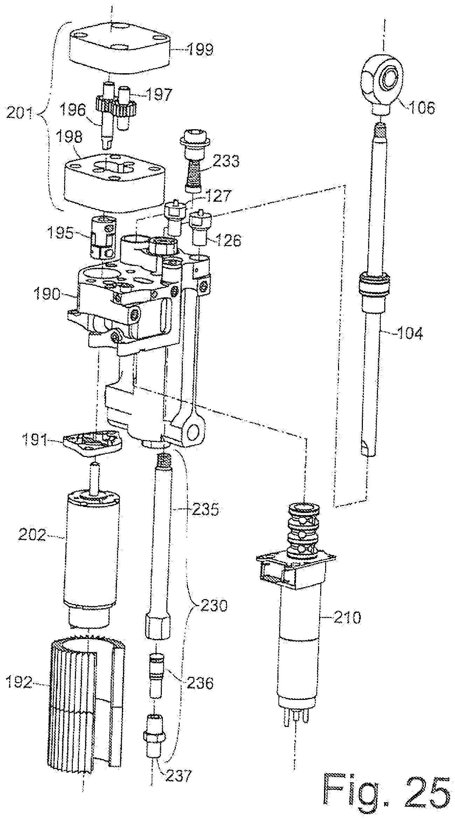

Figure 25 shows the exploded view of hydraulic power unit 200. Hydraulic pump 201 includes a pump cover 199 and a pump base 198. A driver gear 196 is coupled to electric motor 202 through a coupler 195. A driven gear 197 of hydraulic pump 201 is engaged to driver gear 196. Manifold 190 includes all hydraulic passages. Reservoir 230 includes an air/fluid divider 236 and an air valve 237. Air valve 237 allows for pressurizing the air in reservoir 230. A heat sink 192 allows for heat transfer from electric motor 202. Pressure sensors 126 and 127 measure the hydraulic pressure in two chambers of the torque generator 104. A rod end 106 connects torque generator 104 to thigh link 103. Components labeled 191 and 235 are a motor mounting plate and a reservoir housing, respectively.

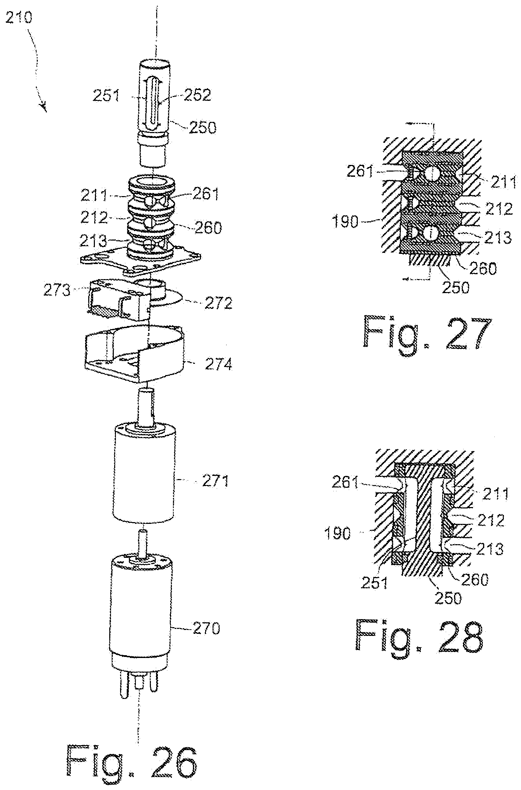

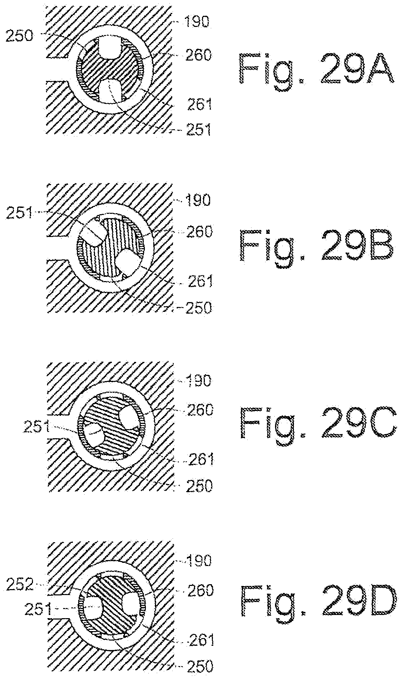

Figure 26 describes the details of three-way valve 210. A valve electric motor 270 is coupled to a valve transmission 271. An encoder, which includes an encoder housing 274, an encoder disk 272 and an encoder read head 273, measures the valve position. A valve housing 260 has three ports 211, 212, and 213. In this embodiment, there are five orifices 261 in valve housing 260. A valve barrel 250 is coupled to valve transmission 271 output shaft. Two slots 251 are created in valve barrel 250 as shown in Figures 26 and 28. As valve barrel 250 is turned by valve electric motor 270, three-way valve 210 assumes one of at least three positions described by Figure 16. As shown in Figure 29A, when three-way valve 210 is in its first position, port 211 and port 213 are fully open to each other. When three-way valve 210 is in its second position (Figure 29B), port 211, port 212 and port 213 are connected. When three-way valve 210 is in its third position (Figure 29C), no ports are connected. As can be seen from Figure 26 and Figure 29D there are some notches 252 on slot 251 that allow for controllable openings of the ports. Needless to say, valve barrel 250 can be in other positions besides positions depicted in Figure 29A-D. To obtain the desired resistance to fluid flow, the valve can be adjusted by signal processor in real time to achieve optimal performance.

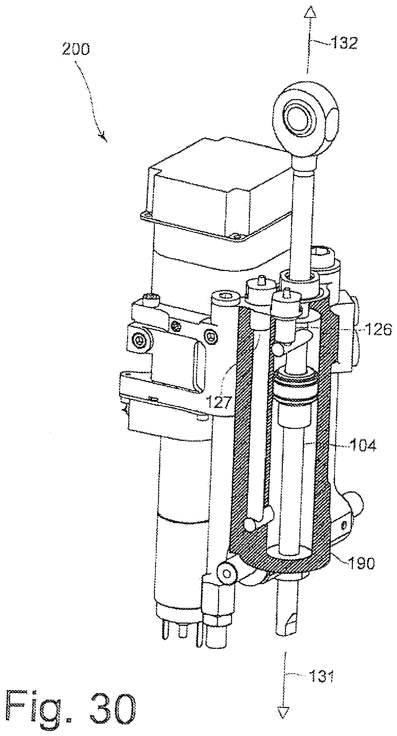

Figure 30 represents an embodiment of semi-actuated prosthetic knee 100 where pressure sensors 126 and 127 measure the hydraulic pressure on both sides of torque generator 104. Additionally, Figure 30 represents an embodiment of hydraulic power unit 200 where hydraulic manifold 190 is shown cut away so that connection paths between torque generator 104 and pressure sensors 126 and 127 are visible.

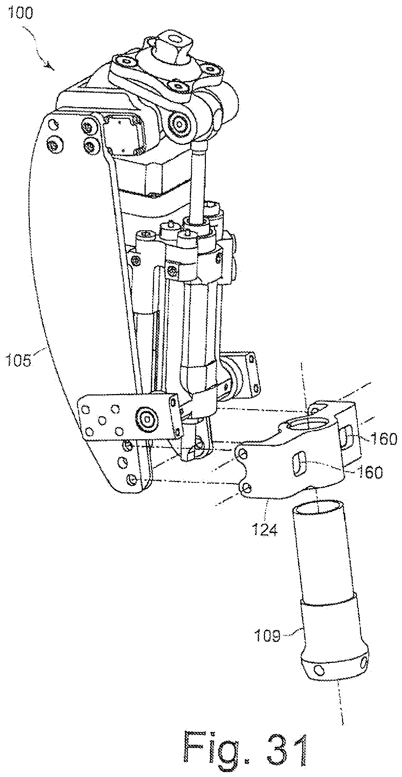

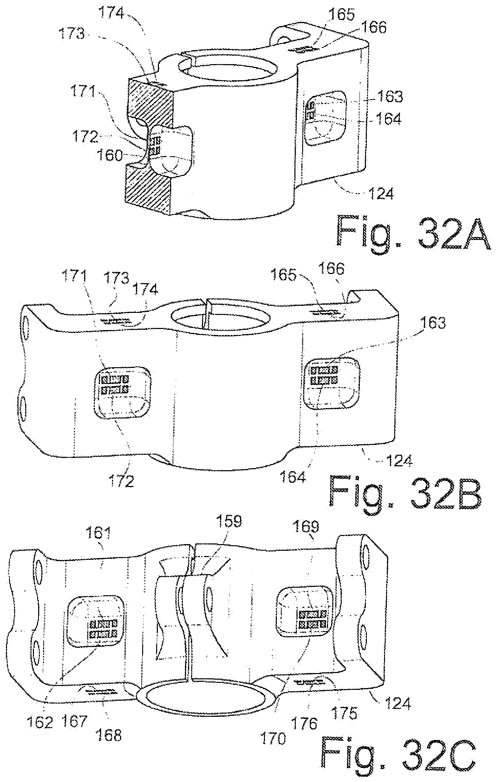

Figure 31 shows the implementation of stance sensor 124 in the embodiment of semi-actuated knee 100 shown in Figure 20. Stance sensor 124 connects ankle pylon 109 to shank link 105. In this embodiment, stance sensor 124 is instrumented with several strain gages 161-172 to measure forces and moments transmitted through shank link 105 during stance phase. Figures 32A-32C shows the locations of strain gages 161-172 on stance sensor 124. Stance sensor 124 comprises a tube clamp 159 as depicted in Figure 32C that clamps to ankle pylon 109.

Strain gages 161, 162, 163, 164 are electrically connected in a wheatstone bridge configuration to measure the vertical shear strains in a shear web 160 due to vertical forces on one of the webs. Strain gages 169, 170, 171, 172 are electrically connected in a wheatstone bridge configuration to measure the vertical shear strain in the second shear web. Summing the vertical shear measurements from both webs 160 cancels out frontal plane moments which might contaminate the vertical shear measurements. Strain gages 165, 166, 167, 168 are electrically connected in a wheatstone bridge configuration to measure the shear strains due to sagittal plane moment loads on the right side of stance sensor 124. Strain gages 173, 174, 175, 176 are electrically connected in a wheatstone bridge configuration to measure the shear strains due to sagittal plane moment loads on the left side of stance sensor 124. Summing the moment load measurements from the left and right sides of stance sensor 124 cancels out rotational moments which might contaminate the sagittal moment measurements. Since rotational moments on stance sensor 124 are small in normal operation in comparison with sagittal plane moments, strain gages 165, 166, 167, 168 or strain gages 173, 174, 175, 176 may be electrically connected in an alternative wheatstone bridge configuration to measure horizontal shear strains due to horizontal forces on the right or left side of stance sensor 124.

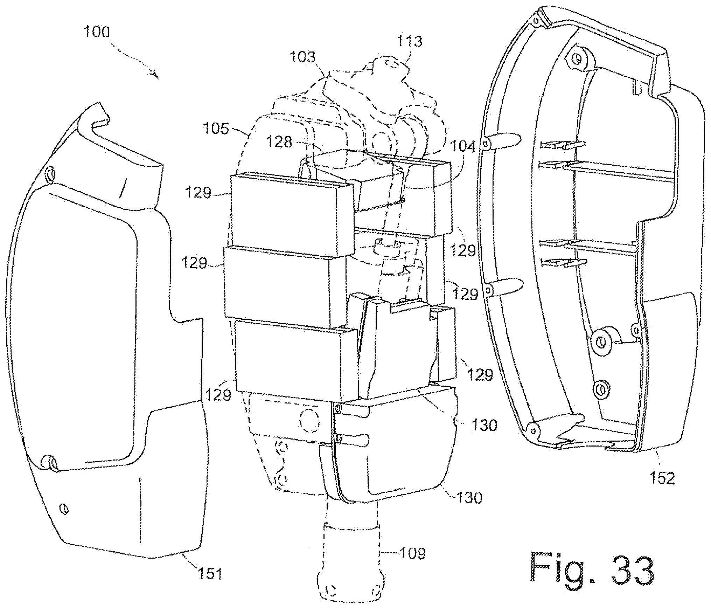

Figure 33 shows semi-actuated prosthetic knee 100 where covers 151 and 152 are removed.

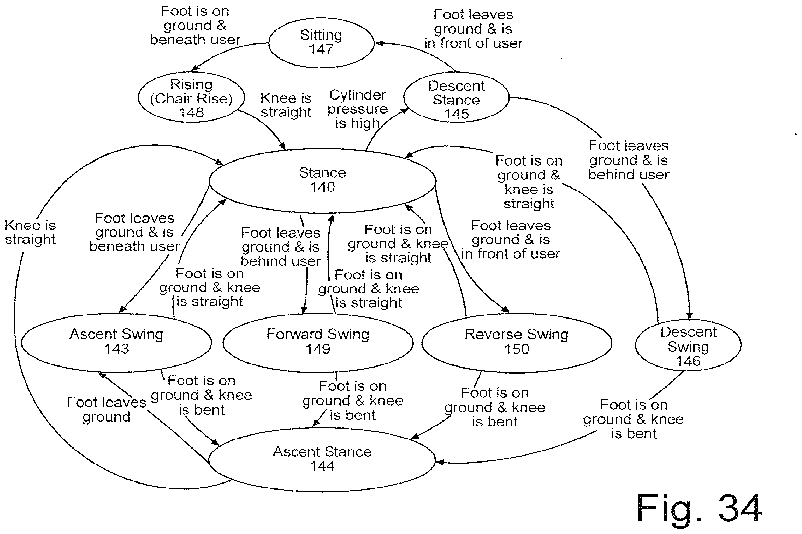

In some embodiments, signal processor 130 receives information from various sensors and implements various controllers onto the knee. These controllers are referred to as "states" in this document. Figure 34 is a diagram of states implemented by signal processor 130. All states are labeled. The arrows show the conditions under which signal processor 130 moves the prosthetic knee from one state to another. Below the states and the conditions to move to that state is described.

### Stance

In operation, signal processor 130 begins to implement a stance state 140 when stance sensor 124 indicates that artificial foot 108 has contacted the ground as depicted in Figure 20. In some embodiments of the invention, during a portion of stance state 140, semi-actuated prosthetic knee 100 operates in the un-actuated mode. This means that during this portion of stance state 140 where semi-actuated prosthetic knee 100 operates in the un-actuated mode, semi-actuated prosthetic knee 100 is configured such that no electric power from electric power source 205 is transferred to electric motor 202 and hydraulic valve circuit 204 modulates the resistance of the fluid flow in torque generator 104. The ability to modulate the resistance of fluid flow in torque generator 104 allows one to control the resistance of knee mechanism 107 to forces and torques during a portion of stance state 140, which reduced use of electric power since electric motor 202 is not consuming any electric power in this un-actuated mode.

In some embodiments of the invention when stance sensor 124 indicates that the heel of artificial foot 108 is taking more load than the toe of artificial foot 108, hydraulic power unit 200 imposes a greater resistance to fluid flow in torque generator 104 than of when stance sensor 124 indicates that the toe of artificial foot 108 is taking more load than the heel of artificial foot 108.

### Forward Swing

In some embodiments of the invention, signal processor 130 begins to implement a forward swing state 149 when semi-actuated prosthetic knee 100 is operating in stance state 140 and signal processor 130 learns that artificial foot 108 has separated from the ground generally behind the amputee's trunk. In some embodiments of the invention, during a portion of forward swing state 149, semi-actuated prosthetic knee 100 operates in the actuated mode. This means during this portion of forward swing 149 where semi-actuated prosthetic knee 100 operates in the actuated mode, semi-actuated prosthetic knee 100 is configured such that it transfers electric power from electric power source 205 to electric motor 202 powering electric motor 202 and hydraulic pump 201. In this actuated mode, hydraulic valve circuit 204 is configured such that hydraulic pump 201 hydraulically couples to torque generator 104 such that the entire hydraulic pump output flow travels to torque generator 104. This hydraulic coupling between hydraulic pump 201 and torque generator 104 allows signal processor 130 to control torque generator 104 directly by controlling electric motor 202. The ability to inject power to torque generator 104 allows one to control the motion of knee mechanism 107 or impose desirable torque onto knee mechanism 107 during a portion or entire forward swing state 149.

In some embodiments of the invention, during a portion of forward swing state 149, signal processor 130 controls the angle between thigh link 103 and shank link 105 such that artificial foot 108 follows a trajectory. In some other embodiments of the invention, during a portion of forward swing state where prosthetic knee 100 operates in the actuated mode, signal processor 130 controls the angle between thigh link 103 and shank link 105 as a function of thigh angle signal 156 (depicted in Figure 1) such that artificial foot 108 follows a trajectory. This allows the amputee to move artificial foot 108 forward and backward (i.e. change direction) during swing and have artificial foot 108 on a trajectory. In some embodiments, the trajectory for artificial foot 108 is a straight line generally parallel to the ground. It should be understood that one can use a shank angle sensor in conjunction with knee angle sensor 120 to arrive at thigh angle signal 156. In more detailed embodiment of the invention, during a portion of forward swing state 149 where prosthetic knee 100 operates in the actuated mode, signal processor 130 controls the angle between thigh link 103 and shank link 105 first as a function of thigh angle signal 156 and then as a function of time. For example in some embodiments, after regulating artificial foot 108 on a trajectory up to a point that artificial foot 108 is in front of the amputee's body, signal processor 130 extends the knee in a time suitable for the current walking speed. In some other embodiments of the invention, during a portion of forward swing state 149 where prosthetic knee 100 operates in the actuated mode, signal processor 130 controls the angle between thigh link 103 and shank link 105 such that the absolute angle of shank link 105 follows a trajectory.

### Reverse Swing

In some embodiments of the invention, signal processor 130 begins to implement a reverse swing state 150 when semi-actuated prosthetic knee 100 is operating in stance state 140 and signal processor 130 learns that artificial foot 108 has separated from the ground in front of the amputee's trunk. In some embodiments of the invention, during a portion of reverse swing state 150, semi-actuated prosthetic knee 100 operates in the actuated mode.

This means that during this portion of reverse swing, the ability to inject power to torque generator 104 allows one to control the motion of knee mechanism 107 or impose desirable torque onto knee mechanism 107 during a portion or entire reverse swing state 150.

In some embodiments of the invention, during a portion of reverse swing state 150, signal processor 130 controls the angle between thigh link 103 and shank link 105 such that artificial foot 108 follows a trajectory. In some other embodiments of the invention, during a portion of reverse swing state 150 where semi-actuated prosthetic knee 100 operates in the actuated mode, signal processor 130 controls the angle between thigh link 103 and shank link 105 as a function of thigh angle signal 156 such that artificial foot 108 follows a trajectory. This allows the amputee to move artificial foot 108 forward and backward (i.e. change direction) during reverse swing 150 and have artificial foot 108 on a trajectory. In some embodiments, the trajectory for artificial foot 108 is a straight line generally parallel to the ground. Again, it should be understood that one can use a shank angle sensor in conjunction with knee angle sensor 120 to arrive at thigh angle signal 156. In a more detailed embodiment of the invention, during a portion of reverse swing state 150 where prosthetic knee 100 operates in the actuated mode, signal processor 130 controls the angle between thigh link 103 and shank link 105 first as a function of thigh angle signal 156 and then as a function of time. For example in some embodiments, after regulating artificial foot 108 on a trajectory up to a point that artificial foot 108 is behind the amputee's body, signal processor 130 extends the knee in a time suitable for walking backwards. In some other embodiments of the invention, during a portion of reverse swing state 150 where prosthetic knee 100 operates in the actuated mode, signal processor 130 controls the angle between thigh link 103 and shank link 105 such that the absolute angle of shank link 105 follows a trajectory.

### Ascent Swing

In some embodiments of the invention, signal processor 130 begins to implement an ascent swing state 143 when semi-actuated prosthetic knee 100 is operating in stance state 140 and signal processor 130 learns that said artificial foot 108 just separated from the ground generally beneath the amputee's trunk. In some embodiments of the invention, during a portion of ascent swing state 143, semi-actuated prosthetic knee 100 operates in the actuated mode. This means during this portion of ascent swing state 143 where semi-actuated prosthetic knee 100 operates in the actuated mode prosthetic knee 100 is configured such that it transfers electric power from electric power source 205 to electric motor 202 turning electric motor 202 and hydraulic pump 201.

In some embodiments of the invention, during a portion of ascent swing state 143, signal processor 130 controls the angle between thigh link 103 and shank link 105 such that artificial foot 108 follows a trajectory. In some other embodiments of the invention, during a portion of ascent swing state signal processor 130 controls the angle between thigh link 103 and shank link 105 as a function of thigh angle signal 156 such that artificial foot 108 follows an arbitrary trajectory. This allows the amputee to move artificial foot 108 up and down (i.e. change direction) during ascent swing and have artificial foot 108 on a trajectory. In some embodiments, the trajectory for artificial foot 108 is a path that moves up and then forward in order to place the artificial foot on top of a stair step. Again, it should be understood that one can use a shank angle sensor in conjunction with knee angle sensor 120 to arrive at thigh angle signal 156. In some other embodiments of the invention, during a portion of ascent swing state 143 where prosthetic knee 100 operates in the actuated mode, signal processor 130 controls the angle between thigh link 103 and shank link 105 such that the absolute angle of shank link 105 follows a trajectory or maintains a constant value.

### Ascent Stance

In some embodiments of the invention, signal processor 130 begins to implement an ascent stance state 144 when stance sensor 124 indicates that artificial foot 108 has contacted the ground with the knee angle substantially bent. During a portion of this ascent stance state 144, semi-actuated prosthetic knee 100 operates in the actuated mode.

In some embodiments of the invention, during a portion of ascent stance state 144, signal processor 130 controls the angle between thigh link 103 and shank link 105 such that the knee angle follows a trajectory. In some other embodiments of the invention, during a portion of ascent stance state 144, signal processor 130 controls the torque generated by torque generator 104. In some further embodiments of the invention, during a portion of ascent stance state 144, signal processor 130 controls the current to electric motor 202. In some other embodiments of the invention, during a portion of ascent stance state 144, signal processor 130 controls the speed of electric motor 202.

In some embodiments of the invention, signal processor 130 begins to implement an ascent swing state 143 when semi-actuated prosthetic knee 100 is operating in ascent stance state 144 and signal processor 130 learns that said artificial foot 108 just separated from the ground (regardless of the position of the foot). Signal processor 130 begins to implement a stance state 140 when semi-actuated prosthetic knee 100 is operating in ascent stance state 144 and knee angle signal 155 indicates that semi-actuated prosthetic knee 100 is not bent.

### Descent Stance

In some embodiments of the invention, signal processor 130 begins to implement a descent stance state 145 when semi-actuated prosthetic knee 100 is operating in stance state 140 and the torque in torque generator 104 is larger than a particular value. During descent stance state 145, the user intends to bend semi-actuated prosthetic knee 100 and that causes an increase in the torque of torque generator 104. In one embodiment, pressure sensors 126 and 127 are used to measure the force in torque generator 104, thereby reflecting the torque associated in torque generator 104. In some embodiments of the invention, signal processor 130 begins to implement a descent stance state 145 when semi-actuated prosthetic knee 100 is operating in stance state 140 and pressure sensors 126 and 127 indicate high pressure difference between first and second torque generator chambers. In some embodiments of the invention, during a portion of descent stance state 145, semi-actuated prosthetic knee 100 operates in the un-actuated mode.

This means during this portion of descent stance state 145 where semi-actuated prosthetic knee 100 operates in the un-actuated mode, semi-actuated prosthetic knee 100 is configured such that no electric power from electric power source 205 is transferred to electric motor 202 and hydraulic valve circuit 204 modulates the resistance of the fluid flow in torque generator 104. The ability to modulate the resistance of fluid flow in torque generator 104 allows one to control the resistance of knee mechanism 107 to forces and torques during a portion of descent stance state 145 with reduced use of electric power since electric motor 202 is not consuming any electric power in this un-actuated mode.

In some embodiments the semi-actuated prosthetic knee 100 includes a power regenerative mode, which is used during descent stance state 145. In this mode, pump valve 203 is not closed allowing at least a portion of the hydraulic flow from torque generator 104 to turn hydraulic pump 201 and the motor controller forces electric motor 202 to generate electric power. This could be accomplished in a number of ways which are not hydraulic as well.

### Descent Swing

In some embodiments of the invention, signal processor 130 begins to implement a descent swing state 146 when signal processor 130 learns that during descent stance state 145 artificial foot 108 just separated from the ground and is positioned behind the amputee's trunk. In some embodiments of the invention, during a portion of descent swing state 145, semi-actuated prosthetic knee 100 operates in the actuated mode.

In some embodiments of the invention, during a portion of descent swing state 145, signal processor 130 controls the angle between thigh link 103 and shank link 105 such that artificial foot 108 follows a trajectory. In some other embodiments of the invention, during a portion of ascent swing state signal processor 130 controls the angle between thigh link 103 and shank link 105 as a function of thigh angle signal 156 such that artificial foot 108 follows a trajectory. In a more detailed embodiment of the invention, during a portion of descent swing state 146 where prosthetic knee 100 operates in the actuated mode, signal processor 130 controls the angle between thigh link 103 and shank link 105 first as a function of thigh angle signal 156 and then as a function of time. For example in some embodiments, after regulating artificial foot 108 on a trajectory up to a point that artificial foot 108 is estimated to have cleared a standard stair, signal processor 130 extends the knee in a time suitable for walking down stairs. In some other embodiments of the invention, during a portion of descent swing state 146 where prosthetic knee 100 operates in the actuated mode, signal processor 130 controls the absolute angle of shank link 105 to follow an arbitrary trajectory.

### Sitting

In some embodiments of the invention, signal processor 130 begins to implement a sitting state 147 when signal processor 130 learns that during descent stance state 145 artificial foot 108 just separated from the ground in front of the amputee's trunk. In some embodiments of the invention, during a portion of sitting state 147, semi-actuated prosthetic knee 100 operates in the un-actuated mode. This means during this portion of sitting state 147 where semi-actuated prosthetic knee 100 operates in the un-actuated mode, semi-actuated prosthetic knee 100 is configured such that no electric power from electric power source 205 is transferred to electric motor 202 and hydraulic valve circuit 204 modulates the resistance of the fluid flow in torque generator 104 so prosthetic knee 100 flexes smoothly with little or no resistance. The ability to modulate the resistance of fluid flow in torque generator 104, allows one to control the resistance of knee mechanism 107 to forces and torques during a portion of stance state 140 with reduced use of electric power since electric motor 202 is not consuming any electric power in this un-actuated mode.

### Rising (Chair Rise)

In some embodiments of the invention, signal processor 130 begins to implement a rising state 148 when stance sensor 124 indicates that, during sitting state 147, artificial foot 108 has contacted the ground beneath the amputee. During a portion of this rising state 148 semi-actuated prosthetic knee 100 operates in the actuated mode. In some embodiments of the invention, during a portion of rise state 148, signal processor 130 controls the angle between thigh link 103 and shank link 105 such that the knee angle follows a trajectory. In some other embodiments of the invention, during a portion of rise state 148, signal processor 130 controls the torque generated by torque generator 104. In some further embodiments of the invention, during a portion of rise state 148, signal processor 130 controls the current to electric motor 202. In some other embodiments of the invention, during a portion of rise state 148, signal processor 130 controls the speed of electric motor 202.

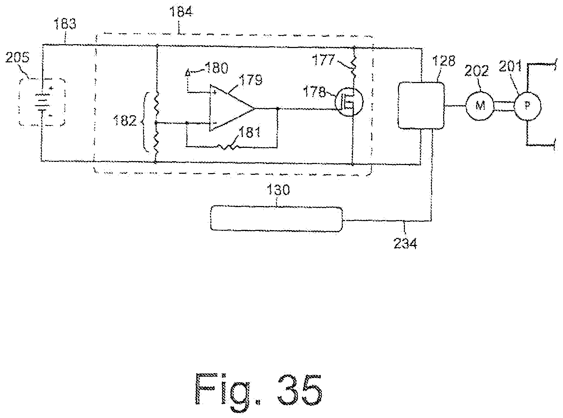

Figure 35 is an electrical schematic showing the connection of electric power source 205 to motor controller 128, including an overcharge protection circuit 184. In power regenerative mode, hydraulic fluid flows through hydraulic pump 201, which causes electric motor 202 to turn and generate electricity. The signal processor 130, commands a desired current to the motor controller 128, which increases the voltage of a bus 183 such that energy flows from the electric motor 202 into the power source 205, thus regenerating power. If the bus voltage becomes sufficiently high, a voltage divider 182 causes a comparator 179 to turn on a switch 178 which diverts regenerating current away from power source 205 and instead dissipates a fraction of the energy in a power resistor 177. A voltage reference 180 sets the trip point for the comparator 179 and a feedback resistor 181 provides hysteresis.

The foot trajectory described above in connection with a prosthesis can be applied to lower extremity human exoskeletons. Just as the prosthetic knee described above controls the knee angle based on the thigh angle signal, in a lower extremity exoskeleton, the motion controller can coordinate the motion of multiple actuated joints using the same technique. As a result the motion controller will need to define multiple constraints for determining the desired joint trajectories. The goal of the technique is to use the coordinated motion of the joints to control the motion of the foot 301 relative to the ground 313. For the purposes of explanation, the description will focus on the embodiment of this device as single leg 309 with controlled hip 308 and knee joints 306. This is in no way meant to limit the applicability of this invention to only lower extremity exoskeletons with two controlled joints. For example, other lower extremity exoskeletons to which this technique appliesarean embodiment with actuated hip, knee and ankle joints or one with actuated hip and knee joints on two legs.

One embodiment of this invention coordinates the hip 308 and knee joints 306 of the swing leg 310 to meet two constraints where at least one of those constraints are Cartesian conditions of the position of the foot301. The constraints that place Cartesian conditions on the foot relative to the ground are referenced as Cartesian constraints. When controlling the position of the foot, the specific embodiment can be configured to focus on any portion of the foot such as but not limited to the toe, the heel, the ball or the ankle of the foot **301.** The point on the foot **301** selected to control the position of will be referred to under the general name of "ground contact point" to encompass these possible embodiments. This is significant because in mobile bipedal robotics, the trajectories for the swing leg **310** are typically planned offline in the form of predefined hip and knee angle trajectory constraints and do not take into account the position of the foot **301** relative to the ground **313.**

The technique of predefining joint angle trajectories has worked well in previous autonomous bipedal robotics because the device has complete control over all aspects of the entire pose of the robot. In exoskeleton devices, the user maintains a significant ability to impact the pose of the device so it must use trajectories that are invariant to the pose the user creates. The inventors have discovered that predefining joint trajectories is difficult to use in a lower extremity exoskeleton because the user can vary the posture of the device with respect to the earth by leaning it backward and forward. That means that a predefined hip and knee angle trajectory may result in the foot **301** striking the ground **313** during mid swing if the user leans the device **309** forward, and it may result in a step terminating in mid air if the user leans the device **309** backwards. Therefore a system that controls the position of the foot **301** with respect to the ground **313** is much easier to use, especially for a novice user. As a result, the presented technique is valuable for allowing a mixture of Cartesian constraints and joint angle constraints that can be predefined without being affected by the pose of the device.

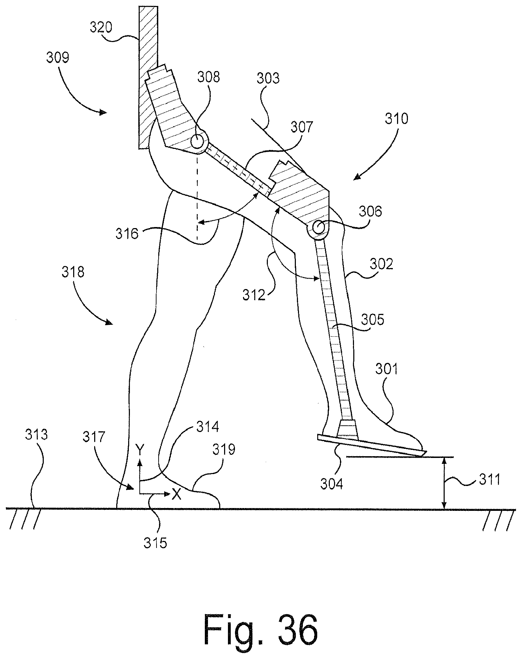

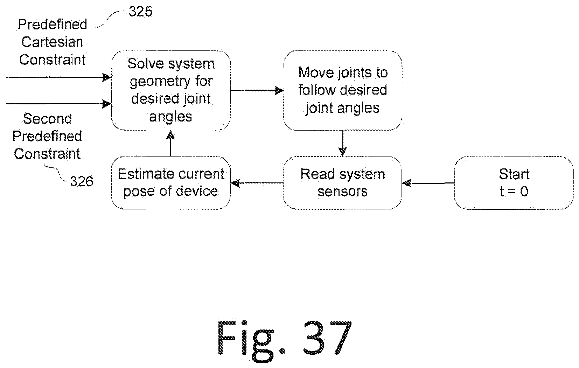

In one such embodiment, the invention can be configured to maintain a desired toe height trajectory relative to the ground and a desired knee angle trajectory as can be seen in Figure 36. To do this the invention calculates a current pose estimate which estimates the positions of the exoskeleton links **305** and **307** with respect to the ground **313.** The motion controller then uses the active feedback to the system provided by the sensors and pose estimate to calculate the specific hip **316** and knee angles **312** required to meet the constraints and moves the hip **308** and knee **306** joints to those positions. Figure 36 shows a predefined Cartesian constraint **325,** the desired toe elevation **311,** and a second predefined constraint **326** is shownin the flow chart of Figure 37. In this case second predefined constraint **326** defines the desired knee angle **312.** The remaining hip angle **316** for the swing leg **310** can then be solved to satisfy these two constraints using many techniques known to one who is skilled in the art. Figure 37 shows the order in which measurements are used to make calculations.

There are many other embodiments of this invention that are similar but incorporate different constraints. Often, it is desirable for the predefined constraints to not be constant and instead to vary throughout the step with respect to time or another step parameter. A set of constraints as it varies throughout the step is referred to as a trajectory. The method equally applies to other embodiments with a combination of vertical **314,** or horizontal **315** Cartesian constraints and knee **312** or hip joint **316** anglesconstraints on the swing leg **310.** A Cartesian constraint is defined as a constraint that describes a Cartesian condition on the position of the foot **301.**

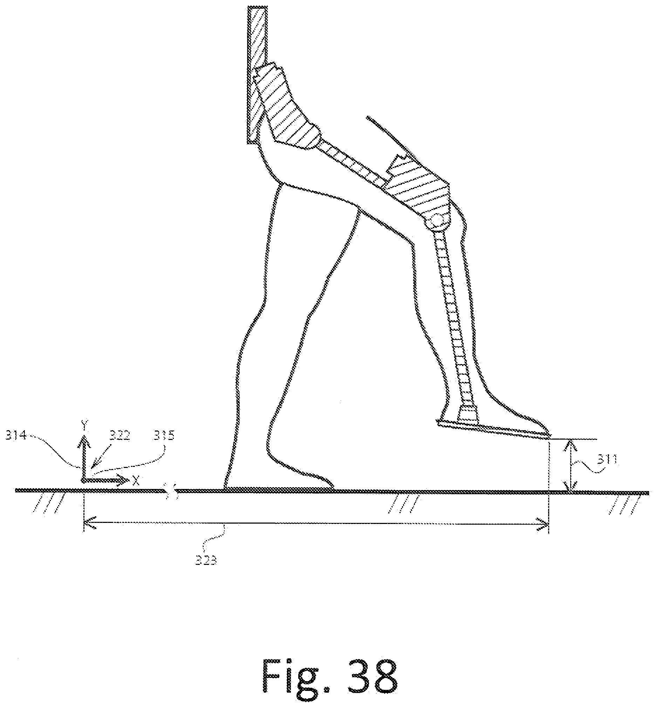

In order to apply this technique, the Cartesian constraints must have a Cartesian coordinate reference. This reference establishes the origin for the Cartesian constraint being used to provide physical context for the constraint. In Figure 36 the Cartesian coordinate reference **317** is defined as a point on the stance foot **319,** indicating that all Cartesian constraints are in reference to the position of the stance foot. Another embodiment of this method could define the Cartesian constraints with respect to an external or global coordinate system **322.** Such an embodiment defines the Cartesian coordinate reference as a fixed point in the surrounding environment of the device **309** as shown in Figure 38 where coordinates **311** and **323** could be defined using a fixed positioning system in the room the device **309** is operating in. Many other embodiments exist that use a wide range of Cartesian coordinate references such as, but not limited to the following: the ground **313** potentially measured directly with a non-contact range sensor attached to the foot **301** or another part of the exoskeleton, a point on thestance leg **318** either on or off the ground **313,** or a point on the torso **320.**

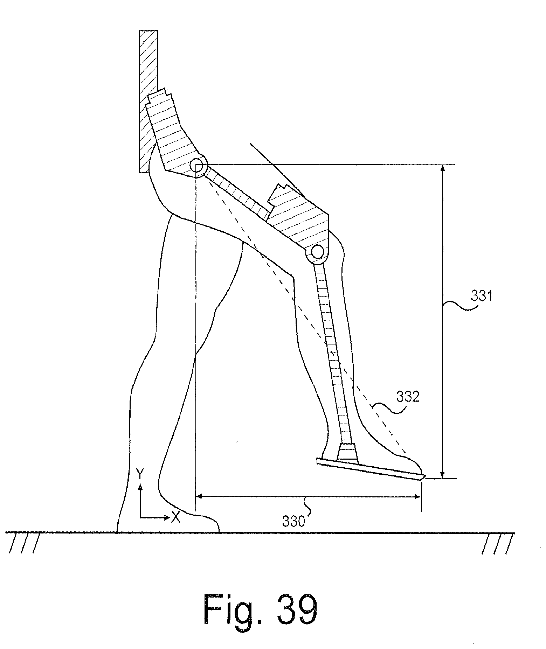

Additional embodiments can also use Cartesian constraints that are not defined strictly in the horizontal **315** or vertical axis **314** but rather are defined as fixing some combination of them both. An example is shown in Figure 39 where horizontal dimension **330** and vertical dimension **331** are controlled such that there is a fixed ratio between them which will result in the toe remaining on the dotted path **332.** Combining this path constraint with a knee angle **312** trajectory will provide two constraints to allow for calculating the remaining swing leg joint angle that meets both constraints. This of course, is just one example of an almost infinite number of relationships that could be defined.

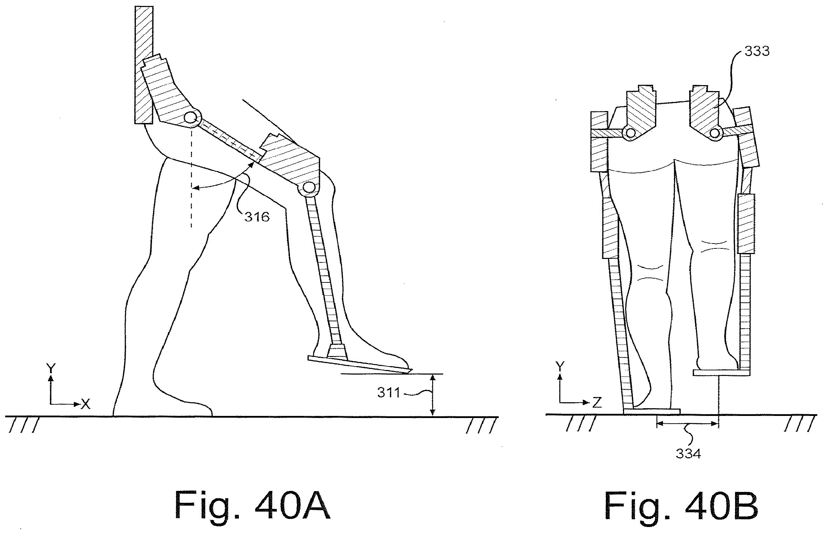

This invention can also extend to embodiments that use more or less than two constraints as long as one of those constraints is a Cartesian constraint. For example, the same method extends to a system that only has one computer controlled joint such as a knee **306** that is attempting to meet the single constraint of maintaining a clearance height **311** of the ground contact point. In this scenario it is necessary that the system has at least as many controllable degrees of freedom as the number of desired constraints they intend to satisfy. As a result, a system with only a controllable knee joint **306** cannot meet both a desired ground contact X **323** and Y **311** constraint because the geometry of the system provides no solvable set to guarantee meeting two constraints on the system. Similarly, the method can apply to systems with more than two constraints as long as there are more controllable degrees of freedom than the number of desired constraints. One such embodiment is a system that has controlled hip **308** and knee joint **306** in the sagittal plane and a controlled hip joint **333** in the frontal plane that seeks to meet three constraints. An example set of constraints for this system could be the ground contact Y coordinate **311**, the hip angle **316** and the frontal plane excursion of the ground contact point **334** as shown in Figure 40. These constraints are a valid set of constraints because they are three independent constraints which fully define the desired geometry of the system.

Another set of embodiments of this invention coordinates the motion of the hip **308** and knee **306** to accomplish a desired foot motion where the constraints change throughout the step. One method for moving between constraints and determining the phase of the swing state is using a finite state machine. These embodiments allow the system to complete a different step depending on how the foot is progressing through the step. These methods can use all of the same constraints presented in the previous embodiments of the invention. In typical applications, the constraints applied when used in a state machine embodiment are in the form of constraint trajectories that are defined with respect to time through the step.

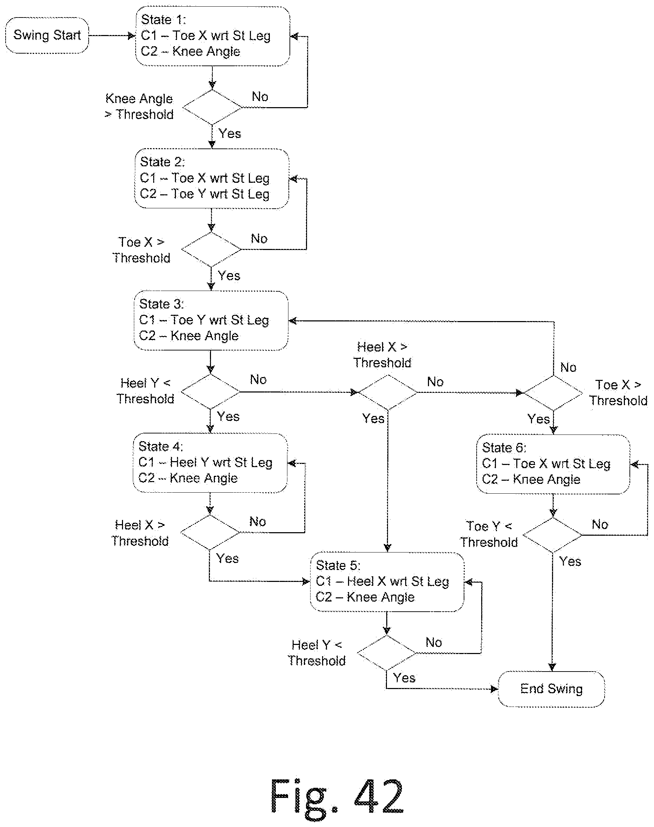

In one embodiment, the system uses a simple finite state machine to define the states of the swing based on their differing constraints as shown inFigure 41. In the first state **340,** the swing leg **310** is intended to buckle and prepare to swing through. This can be defined with constraints of the toe X coordinate **323** relative to a point on the stance leg **318** and the swing knee angle **312** trajectory. The transition to the next state **343** can be defined in many ways, but in this example the transition is happening at a predefined knee angle **312** threshold.In the second state **341,** the swing leg **310** progresses the foot **301** through the minimum clearance stage. This motion can be defined using constraints of the toe X coordinate **323** and the toe Y coordinate **311** relative to a point on the stance leg **318** (a simple path in x, y space). After the minimum clearance stage is complete, this state transitions to the next state **344** based on a set toe X coordinate **323** relative to a point on the stance leg **318.** In the third state **345,** the swing leg **310** prepares for heelstrike using constraints on the heel X coordinate relative to a point on the stanceleg and the knee angle **312** trajectory. This can be expanded to a more complex embodiment of the invention that incorporates more states to better isolate the desired behavior of the foot **301** at any one time as shown in Figure 42.

In general, the invention should only be limited by the scope of the claims.

## Claims

1. A powered lower extremity orthotic, configured to be coupled to first and second lower limbs of a person, comprising, for each of said first and second lower limbs, a respective first and second leg including:
a shank link (105; 305) configured to be attached to a person;
a thigh link (103; 307) configured to be attached to a person;
a torso link (320) configured to be attached to a person;
a knee mechanism (107; 306) interconnecting said thigh link and said shank link, said knee mechanism allowing flexion and extension movements of said thigh link and said shank link relative to each other;
a knee torque generator (104) configured to generate torque between said shank link and said thigh link;
a knee angle sensor (120) creating a knee angle signal representing an angle between said shank link and said thigh link;
a hip mechanism (308) interconnecting said thigh link and said torso link, said hip mechanism allowing flexion and extension movements of said thigh link and said torso link relative to each other;
a hip torque generator configured to generate torque between said shank link and said torso link;
a hip angle sensor creating a hip angle signal representing an angle between said torso link and said thigh link; and
a power source configured to provide electric power;
a signal processor connected to the power source, the knee torque generator, the hip torque generator, the knee angle sensor and the hip angle sensor for each of said first and second legs, wherein said signal processor, for each of said first and second legs, receives signals from the knee and hip sensors, and controls the angles between said shank link and said thigh link and between said thigh link and said torso link through the knee torque and hip torque generators respectively such that a reference point on the first leg (310) not touching a ground surface follows a predetermined trajectory that is defined by at least one Cartesian coordinate,
**characterized in that** a position of said reference point being determined from an orientation of the second leg (318) which is in contact with the ground surface.

2. A powered lower extremity orthotic as claimed in claim 1, wherein the Cartesian coordinate is the height of the reference point from the ground surface.

3. A powered lower extremity orthotic as claimed in claim 1, further comprising, for each of the first and second legs:
an artificial foot (108; 301) having a toe and a heel, with the artificial foot being coupled to a respective said shank link;
wherein said signal processor determines that the first leg is in a swing state and controls the angles between said shank link and said thigh link and between the thigh link and the torso link through the knee and hip torque generators respectively such that said artificial foot of the first leg follows the predetermined trajectory.

4. A powered lower extremity orthotic as claimed in claim 3, wherein the predetermined trajectory is generally parallel to the ground surface.

## Patentansprüche

1. Mit Strom betriebene Orthese für die unteren Extremitäten, die so konfiguriert ist, dass sie an die ersten und zweiten unteren Extremitäten einer Person gekoppelt werden kann, die für jede der ersten und zweiten unteren Extremitäten jeweils ein erstes und zweites Bein umfasst, einschließend:
ein Unterschenkelelement (105; 305), das konfiguriert ist, um an einer Person befestigt zu werden;
ein Oberschenkelelement (103; 307), das konfiguriert ist, um an einer Person befestigt zu werden;
ein Rumpfelement (320), das konfiguriert ist, um an einer Person befestigt zu werden;
einen Kniemechanismus (107; 306), der das Oberschenkelelement und das Unterschenkelelement miteinander verbindet, wobei der Kniemechanismus Beugungs- und Streckbewegungen des Oberschenkelelements und des Unterschenkelelements relativ zueinander ermöglicht;
einen Kniedrehmomentgenerator (104), der konfiguriert ist, um ein Drehmoment zwischen dem Unterschenkelelement und dem Oberschenkelelement zu generieren;
einen Kniewinkelsensor (120), der ein Kniewinkelsignal erzeugt, das einen Winkel zwischen dem Unterschenkelelement und dem Oberschenkelelement darstellt;
einen Hüftmechanismus (308), der das Oberschenkelelement und das Rumpfelement miteinander verbindet, wobei der Kniemechanismus Beugungs- und Streckbewegungen des Oberschenkelelements und des Rumpfelements relativ zueinander ermöglicht;
einen Hüftdrehmomentgenerator, der konfiguriert ist, um ein Drehmoment zwischen dem Unterschenkelelement und dem Rumpfelement zu generieren;
einen Hüftwinkelsensor, der ein Hüftwinkelsignal erzeugt, das einen Winkel zwischen dem Rumpfelement und dem Oberschenkelelement darstellt; und
eine Stromquelle, die konfiguriert ist, um elektrischen Strom bereitzustellen;
einen Signalprozessor, der mit der Stromquelle, dem Kniedrehmomentgenerator, dem Hüftdrehmomentgenerator, dem Kniewinkelsensor und dem Hüftwinkelsensor für jeden der ersten und zweiten Beine verbunden ist, wobei der Signalprozessor für jeden der ersten und zweiten Beine Signale von den Knie- und Hüftsensoren empfängt, und die Winkel zwischen dem Unterschenkelelement und dem Oberschenkelelement sowie zwischen dem Oberschenkelelement und dem Rumpfelement über die Knie- bzw. Hüftdrehmomentgeneratoren so steuert, dass ein Referenzpunkt auf dem ersten Bein (310), das keine Bodenoberfläche berührt, einer vorbestimmten Bewegungsbahn folgt, die durch mindestens eine kartesische Koordinate definiert ist,
**dadurch gekennzeichnet, dass** eine Position des Referenzpunktes aus einer Ausrichtung des zweiten Beins (318) bestimmt wird, das in Kontakt mit der Bodenoberfläche steht.

2. Mit Strom betriebene Orthese für die unteren Extremitäten nach Anspruch 1, wobei die kartesische Koordinate die Höhe des Referenzpunktes von der Bodenoberfläche ist.

3. Mit Strom betriebene Orthese für die unteren Extremitäten nach Anspruch 1, jeweils für das erste und das zweite Bein weiter umfassend:
einen künstlichen Fuß (108; 301), der eine Zehe und eine Ferse aufweist, wobei der künstliche Fuß mit einem entsprechenden Unterschenkelelement verbunden ist;
wobei der Signalprozessor bestimmt, dass sich das erste Bein in einem Schwingungszustand befindet, und die Winkel zwischen dem Unterschenkelelement und dem Oberschenkelelement und zwischen dem Oberschenkelelement und dem Rumpfelement durch die Knie- bzw. Hüftdrehmomentgeneratoren so steuert, dass der künstliche Fuß des ersten Beins der vorbestimmten Bewegungsbahn folgt.

4. Mit Strom betriebene Orthese für die unteren Extremitäten nach Anspruch 3, wobei die vorbestimmte Bewegungsbahn im Allgemeinen parallel zur Bodenoberfläche verläuft.

## Revendications

1. Orthèse électrique des membres inférieurs, configurée pour être couplée aux premier et second membres inférieurs d'une personne, comprenant, pour chacun desdits premier et second membres inférieurs, une première et une seconde jambe respective incluant :
un lien pour membre inférieur (105 ; 305) configuré pour être fixé à une personne ;
un lien pour cuisse (103 ; 307) configuré pour être fixé à une personne ;
une articulation torse (320) configurée pour être fixée à une personne ;
un mécanisme de genou (107 ; 306) reliant ledit lien pour cuisse et ledit lien pour membre inférieur, ledit mécanisme de genou permettant des mouvements de flexion et d'extension dudit lien pour cuisse et dudit lien pour membre inférieur l'un par rapport à l'autre ;
un générateur de couple de genou (104) configuré pour générer un couple entre ledit lien pour membre inférieur et ledit lien pour cuisse ;
un capteur d'angle de genou (120) créant un signal d'angle de genou représentant un angle entre ledit lien pour membre inférieur et ledit lien pour cuisse ;
un mécanisme de hanche (308) reliant ledit lien pour cuisse et ladite articulation torse, ledit mécanisme de hanche permettant des mouvements de flexion et d'extension dudit lien pour cuisse et de ladite articulation torse l'un par rapport à l'autre ;
un générateur de couple de hanche configuré pour générer un couple entre ledit lien pour membre inférieur et ladite articulation torse ;
un capteur d'angle de hanche créant un signal d'angle de hanche représentant un angle entre ladite articulation torse et ledit lien pour cuisse ; et
une source d'alimentation électrique configurée pour fournir un courant électrique ;
un processeur de signaux raccordé à la source d'alimentation électrique, le générateur de couple de genou, le générateur de couple de hanche, le capteur d'angle de genou et le capteur d'angle de hanche pour chacun desdites première et seconde jambes, dans lequel ledit processeur de signaux, pour chacun desdites première et seconde jambes, reçoit des signaux provenant des capteurs de genou et de hanche, et commande les angles entre ledit lien pour membre inférieur et ledit lien pour cuisse et entre ledit lien pour cuisse et ladite articulation torse à travers, respectivement, les générateurs de couple de genou et de couple de hanche de sorte qu'un point de référence sur la première jambe (310) ne touchant pas la surface du sol suit une trajectoire prédéterminée qui est définie par au moins une coordonnée cartésienne,
**caractérisée en ce qu'**une position dudit point de référence étant déterminée à partir d'une orientation de la seconde jambe (318) qui est en contact avec la surface du sol.

2. Orthèse électrique des membres inférieurs selon la revendication 1, dans laquelle la coordonnée cartésienne est la hauteur du point de référence à partir de la surface du sol.

3. Orthèse électrique des membres inférieurs selon la revendication 1, comprenant en outre, pour chacun des première et seconde jambes :
un pied artificiel (108 ; 301) présentant une pointe et un talon, avec le pied artificiel étant couplé à un dit lien pour membre inférieur respectif ;
dans laquelle ledit processeur de signaux détermine si la première jambe est dans un état oscillant et commande les angles entre ledit lien pour membre inférieur et ledit lien pour cuisse et entre le lien pour cuisse et l'articulation torse à travers, respectivement, les générateurs de genou et de hanche de sorte que ledit pied artificiel de la première jambe suit la trajectoire prédéterminée.

4. Orthèse électrique des membres inférieurs selon la revendication 3, dans laquelle la trajectoire prédéterminée est généralement parallèle à la surface du sol.
